# EUROPÄISCHE PATENTSCHRIFT

(11) **EP 0 582 244 B1**
(45) Veröffentlichungstag und Bekanntmachung des Hinweises auf die Patenterteilung: **31.10.2001**
(21) Anmeldenummer: 93112338.4
(22) Anmeldetag: 02.08.1993
(51) Int. Cl.: C12N 1/16, C12N 15/81

(54) **Hefewirtsstämme mit Defekten in der N-Glycosylierung**
Yeast strains with impaired N-glycosylation
Soucher de levure avec N-glycosylation défectueuse

(30) Priorität: 07.08.1992 DE 4226094; 25.01.1993 DE 4301932
(43) Veröffentlichungstag der Anmeldung: 09.02.1994
(73) Patentinhaber: Roche Diagnostics GmbH, 68298 Mannheim (DE)
(72) Erfinder: Lehle, Ludwig, Prof. Dr., D-93051 Regensburg (DE); Lehnert, Klaus, Dr., D-63546 Hammersbach (DE); Kopetzki, Erhard, Dr., D-82377 Penzberg (DE)
(74) Vertreter: Schreiner, Siegfried, Dr.

(56) Entgegenhaltungen:
- EP-A- 0 314 096
- EP-A- 0 415 565
- WO-A-89/12675
- JOURNAL OF BIOLOGICAL CHEMISTRY., Bd.257, Nr.6, 1982, BALTIMORE US Seiten 3203 - 3210 HUFFAKER T.C. ET AL. 'Temperature-sensitive yeast mutant...'
- YEAST, Bd.8, Nr.7, Juli 1992, CHICHESTER UK Seiten 535 - 547 NAGASU T ET AL 'ISOLATION OF NEW TEMPERATURE-SENSITIVE MUTANTS OF SACCHAROMYCES-CEREVISIAE DEFICIENT IN MANNOSE OUTER CHAIN ELONGATION'
- MOLECULAR AND CELLULAR BIOLOGY, Bd.1, Nr.10, 1981, WASHINGTON Seiten 902 - 909 HIRSCHBERG C.B. ET AL. 'SELECTION OF MUTANT...'

## Beschreibung

Gegenstand der Erfindung sind Hefewirtsstämme mit Defekten in der N-Glycosylierung sowie deren Verwendung zur Expression von einheitlich glycosylierten Proteinen.

Ein Protein kann auf 3 Arten posttranslational mit Kohlenhydraten versehen werden. Man unterscheidet zwischen:
* N-Glycosylierung
   - N-glycosidische Bindung der Kohlenhydratkette zu Asn
* O-Glycosylierung
   - O-glycosidische Bindung der Kohlenhydratkette zu Thr oder Ser
* Glycosyl-phosphiatidyl-inositolanker (GPI)
   - Bestandteil einiger Membranproteine,
   - Der GPI-Anker dient zur Einlagerung in die Phospholipidmembran.

Die Glycosylierung von Proteinen ist beispielsweise beschrieben in:
- Kukuruzinska, M.A. et al., Ann. Rev. Biochem. 56 (1987) 915-944;
- Paulson, C.P., TIBS 14 (1989) 272-276;
- Warren, C.E., BFE 7 (1990) 392-395;
- Ballou, C.E., In: Strathern, J.N., et al., The Molecular Biology of the Yeast Saccharomyces, Cold Spring Harbor Laboratory, New York, pp. 355-360 (1982).
- Kornfeld, R.; Kornfeld, S., Ann. Rev. Biochem 54 (1985) 631-664;
- Tanner, W.; Lehle, L., Biochim. Biophys. Acta 906 (1987) 81-99;
- Innis, M.A., In: Barr, P.J. et al., Yeast genetic engineering, Butterworths, Stoneham, Mass, pp. 233-246 (1989).

Die O-glycosidischen Kohlenhydratstrukturen von Hefeproteinen bestehen aus einer unverzweigten Mannosekette von 1 - 5 Mannoseresten. Die O-Glycosylierung beginnt im ER (Transfer des ersten Mannoserestes) und wird im Golgi Apparat beendet.

Die N-Glycosylierung erfolgt in 2 Schritten. An einem Lipidcarrierintermediat wird eine "core" Einheit aus N-Acetylglucosamin, Mannose und Glucose aufgebaut, die im ER auf Asn-Reste von "Glycoproteinen" übertragen wird. Nach Prozessierung der Protein-gebundenen "core"-Einheit (Abspaltung der Glucosereste und eines spezifischen Mannoserestes im ER) wird im Golgi Apparat die Zuckerstruktur verlängert ("outer chain" Glycosylierung). Die Struktur der "outer chain" Glycosylierung ist Organismus spezifisch.

Die "outer chain" Glycosylierung von sezernierten Hefeproteinen ist vom "high" Mannose Typ, d. h. besteht aus einer langen Polymannose-Oligosaccharidkette. Heterolog in Hefe sezernierte Proteine werden ebenfalls mit dieser Hefe-spezifischen "outer chain" Glycosylierung vom "high" Mannose Typ versehen, die auch als Hyperglycosylierung bezeichnet wird. Sie ist in vielen Fällen unerwünscht, da dabei ein heterogenes Protein-Produkt (Kohlenhydratanteil, Molekulargewicht) entsteht. Zudem kann die Proteinaufreinigung durch den heterogenen Kohlenhydratanteil erschwert werden. Die Hyperglycosylierung kann die posttranslationale Prozessierung (z.B. Reifung eines "prepro" Proteins zum nativen Protein durch proteolytische Abspaltung des prepro Segments) aus sterischen Gründen verhindern bzw. die Spaltungseffizienz herabsetzen (Bekkers, A.C.A.P.A. et al., Biochim. Biophys. Acta 1089 (1991) 345-351). Weiterhin ist die spezifische Aktivität (in Units/Gewichtseinheit) hyperglycosylierter Enzyme durch den erhöhten Kohlenhydratanteil erniedrigt. Zudem ist die Hefe spezifische "outer chain" Glycosylierung stark immunogen, was bei therapeutischer Anwendung unerwünscht ist.

Die Glucoseoxidase (GOD) aus Aspergillus niger ist ein natürlich sezerniertes N-glycosyliertes Homodimer (Molekulargewicht/Untereinheit (UE): ca. 80 kDa, Cofaktor: 1 FAD/UE, 1 SS-Brücke/UE). Heterolog in Saccharomyces cerevisiae exprimierte GOD wird sehr effizient ins Medium sezerniert. Das Enzym ist enzymatisch aktiv, jedoch durch eine nicht einheitliche "outer chain" Glycosylierung von bis zu 150 Mannoseresten hinsichtlich des Kohlenhydratanteils und des Molekulargewichts heterogen. Die aus A. niger isolierte GOD besitzt im Gegensatz dazu eine relativ einheitliche Kohlenhydratstruktur ("core" Glycosylierung).
- Kriechbaum, M. et al., FEBS Lett. 255 (1989) 63-66;
- Frederick, K.R. et al., J. Biol. Chem. 265 (1990) 3793-3802;
- De Baetselier, A. et al., Biotechnology 9 (1991) 559-561;
- Whittington, H. et al., Curr. Genet. 18 (1990) 531-536;
- Rosenberg, S., WO 89/12675;

Außerdem hat die in Hefe exprimierte/sezernierte GOD durch Hyperglycosylierung eine geringere spezifische Aktivität (Units/g Enzym) im Vergleich zu dem aus A. niger isolierten Enzym. Die in A. niger sezernierte GOD besitzt eine relativ einheitliche Kohlenhydratstruktur ("core ähnliche" Glycosylierung, Molekulargewicht/UE: ca. 80 kDa).

Auch die N-Glycosylierung sezernierter Hefeproteine ist meist nicht einheitlich. Dies ist beispielsweise für die externe S. cerevisiae Invertase bekannt (Reddy, V.A. et al., J. Biol. Chem. 263 (1988) 6978-6985; Ziegler, F.D. et al., J. Biol. Chem. 263 (1988) 6978-6985). Von den 14 potentiellen Sequons (Sequon, Glycosylierungsstelle, Aminosäure Sequenzmotiv: Asn-X-Ser/Thr) der Invertase werden 13 vollständig oder partiell glycosyliert. Von den 13 genutzten Sequons werden jedoch pro Invertase Untereinheit im Durchschnitt nur 9 - 10 glycosyliert. Ein durch die Proteinsequenz definiertes Sequon kann
i) immer glycosyliert,
ii) nie glycosyliert oder
iii) nur manchmal glycosyliert werden.
Weiterhin besitzt ein definiertes Sequon entweder eine kurze Oligosaccharidkette (GlcNAc₂Man₈₋₁₅) oder eine lange Polymannose-Oligosaccharidkette (GlcNAc₂Man₅₀₋ ₁₀₀). Die beobachtete heterogene N-Glycosylierung der Invertase ist bedingt durch:
i) nur teilweise Glycosylierung der potentiell genutzten Sequons pro Molekül (z.B. es werden nur 3 von 5 potentiell genutzten Sequons rein zufällig glycosyliert),
ii) Vorhandensein von kurzen "core"-Oligosacchariden und langen Polymannoseketten (outer chain) und,
iii) Variation der "outer chain" Kettenlänge.

Um Glycoproteine mit reduziertem bzw. fehlendem Kohlenhydratanteil, also mit einheitlichem Kohlenhydratanteil zu erhalten, sind folgende Vorgehensweisen bekannt:
* Expression des Glycoprotein kodierenden Gens in Gegenwart von Inhibitoren der Glycosylierung (z.B. Tunicamycin) bzw. des Vesikeltransports (z.B. Brefeldin A)
* Enzymatische Deglycosylierung der Proteine in vitro z.B. mit Endo F oder/und Endo H oder/und N-Glycosidase F
* Entfernung/Veränderung von Glycosylierungsstellen durch Mutagenese auf DNA Ebene
* Verwendung von glycosylierungsdefekten Wirtsstämmen

Hefemutanten mit veränderter N-Glycosylierung sind bekannt. Dabei unterscheidet man zwischen Sekretionsmutanten mit blockierter Sekretion und Mutanten, die Proteine mit veränderter N-Glycosylierung sezernieren.

Sekretionsmutanten besitzen einen lokalisierten Block in der Sekretionsmaschinerie, wodurch unvollständig N-glycosylierte Proteine in dem entsprechenden Zellkompartiment akkumulieren.
z.B.:
* sec Mutanten ("secretion defective") von R. Schekman,
   - Novick, P. et al., Cell 21 (1980) 205-215;
   - Schekman, R. und Novick, P., In: Strathern, J.N. et al., The Molecular Biology of the Yeast Saccharomyces. Cold Spring Harbor Laboratory, New York, pp. 361-398 (1982);
* bet Mutanten ("blocked early in transport") von S. Ferro-Novick,
   - Ferro-Novick, S. und Newman A.P., J. Cell. Biol. 105 (1987) 1587-1594;
* ypt1 Mutante von D. Gallwitz,
   - Schmitt, H.D. et al., Cell 47 (1986) 401-412;
   - Schmitt, H.D. et al., Cell 53 (1988) 635-647;
* sarl Mutante von M. Muramatsu,
   - Nakano, A. und Muramatsu, M., J. Cell. Biol. 109 (1989) 2677-2691.

N-glycosylierungsdefekte Mutanten verfügen in der Regel über einen funktionsfähigen Sekretionsweg. Defekte N-Glycosylierung kann auf unterschiedlichen Gendefekten beruhen, wie z.B. Mutationen
* im Kohlenhydrat-aufbauenden (modifizierenden) Enzymsystem,
* im zellulären Proteintransportsystem ("sorting, targeting").
Bei "sorting" und "targeting" Mutanten werden bei der Sekretion von Proteinen z.B. der Golgi Apparat bzw. Teilbereiche des Golgi Apparates in einem "bypass" umgangen, das Zellkompartiment, in dem unter anderem die Reaktionen der "outer chain" Glycosylierung stattfinden.
* mnn Mutanten ("mannan defective") von C.E. Ballou,
   - Ballou, L. et al., J. Biol. Chem. 255 (1980) 5986-5891;

   - Ballou, C.E., Methods Enzymol. 185 (1990) 440-470;
   - Ballou, C.E., In: Strathern, J.N. et al., The Molecular Biology of the Yeast Saccharomyces. Cold Spring Harbor Laboratory, New York, pp. 355-360 (1982);
* vrg Mutanten ("vanadat resistant glycosylation") von C.E. Ballou,
   - Ballou, L. et al. Proc. Natl. Acad. Sci. 88 (1991) 3209-3212;
* alg Mutanten ("asparagine-linked glycosylation defective) von R. Robbins,
   - Huffaker, T.C. und Robbins, P.W., Proc. Natl. Acad. Sci. 80 (1983) 7466-7470;
   - Runge, K.W. und Robbins, P.W., In: Bonventre, P.F. et al., Microbiology, American Society for Microbiology, Washington, D.C. pp. 312-316 (1986);
* pmr1 (ssc1) Mutante von G. Fink, (D.T. Moir),
   - Duncan, M.J. und Smith, R.A., EPA 0211208;
   - Fink, G.R., EPA 0382332;
   - Rudolph, H.K. et al., Cell 58 (1989) 133-145;
* erdl Mutante ("endoplasmatic reticulum retention defective") von H.R.B. Pelham,
   - Hardwick, K.G. et al., EMBO J. 9 (1990) 632-630.

Mutanten mit einem Block im Sekretionsweg sind für biotechnologische Zwecke (homologe und heterologe Sekretion von Proteinen) verständlicherweise nicht geeignet.

Eine Vielzahl der N-glycosylierungsdefekten Mutanten sind konditional lethal, d.h. sie sind unter normalen Bedingungen (Anzuchttemperatur 30°C) nicht lebensfähig und besitzen einen Temperatur-sensitiven (ts) Phänotyp, wie z.B. die Mutanten alg1, alg2, alg4, bet1, bet2, fast alle sec Mutanten und ypt1 ("cold sensitive").

Ein Temperatur-sensitiver Phänotyp bedeuted, daß die lethale ts-Mutation erst nach einem Temperatur-Shift von z.B. 26°C (permissive Wachstumsbedingungen) auf 37°C (nicht-permissive Wachstumsbedingungen) zur Ausprägung kommt. Auch diese Mutanten sind verständlicherweise für biotechnologische Zwecke weniger geeignet.
Eine Mutantengruppe, die durch eine stark reduzierte (mnn7, mnn8, mnn10) bis fast vollständig fehlende "outer chain" Glycosylierung bzw. modifizierte "core" Glycosylierung (mnn9) charakterisiert ist, besitzt folgende Nachteile: Das Zellwachstum ist verlangsamt, die Zellen sind morphologisch verändert und lysieren bereits während der Anzucht, so daß diese Mutanten nur in osmotisch stabilisierten Medien angezogen werden können (Zusatz von ca. 0.5 M KCl bzw. ca. 1 M Sorbit).

Zudem weisen einige der in der Literatur beschriebenen N-glycosylierungsdefekten Mutanten (z.B. alg1) einen Defekt auf, der nur teilweise in der Zelle ausgeprägt ist ("leaky"). Dies führt zu einer heterogenen N-Glycosylierung ("Kontamination", Überlagerung durch Wildtyp N-Glycosylierung).

Hefestämme, die in der Lage sind, Proteine mit verkürzter bzw. fehlender "outer chain" N-Glycosylierung zu exprimieren/sekretieren sind beispielsweise beschrieben in der EP-A 0 344 864. Die in dieser Anmeldung beschriebenen Stämme beruhen alle auf der von C.E. Ballou beschriebenen mnn9 Mutation. Mnn9 Mutantenstämme neigen zur Lyse während des Wachstums und müssen deshalb osmotisch stabilisiert werden (siehe oben).

Die in der EP-A 0 314 096 beschriebenen Hefestämme basieren ebenfalls auf der mnn9 Mutation. Die Stämme wurden hinsichtlich Lyseempfindlichkeit so verbessert, daß auf osmotische Stabilisatoren im Medium verzichtet werden kann.

Dazu wurde das MNN9 Gen kloniert und anschließend Stämme konstruiert, in denen des MNN9 Gen unter der Kontrolle eines "von außen" regulierbaren Promotors steht. Dadurch wird erreicht, daß z.B. während der Anzuchtphase der Zellen genügend aktives MNN9 Genprodukt zur Synthese notwendiger zellulärer glycosylierter Wirtsproteine vorhanden ist und nur in der eigentlichen Produktionsphase des gewünschten N-hypoglycosylierten Proteins die mnn9 Mutation zur Ausprägung kommt.

Bei dieser Methode bestehen jedoch folgende Nachteile:
* Die Zellkultivierung ist wesentlich komplizierter.
* Nach Erreichen der gewünschten Zelldichte muß das MNN9 Gen z.B. durch einen Temperatur-Shift der Kultur abgeschaltet werden, bevor die Synthese des gewünschten N-hypoglycosylierten Proteins induziert wird.
* Nach Abschaltung des MNN9 Gens besitzen die Zellen den mnn9 Phänotyp, d.h. wachsen schlecht usw. (siehe oben).
* Noch vorhandenes aktives MNN9 Genprodukt kann trotz Abschaltung des MNN9 Gens bei bereits induzierter Produktsynthese zur Synthese geringer Mengen des nicht erwünschten hyperglycosylierten Proteinproduktes führen.

Es ist weiter bekannt, daß Hefesekretionsmutanten (Supersekretionsmutanten) oft hypoglycosylierte Glycoproteine sezernieren. Ein solches Beispiel ist in der EP-A 0 382 332 beschrieben.

Die molekulare Ursache der ssc1 (pmr1) Mutation basiert sehr wahrscheinlich auf der Inaktivierung einer D-Typ ATPase, die am Vesikeltransport zwischen ER und Golgi Komplex beteiligt ist. Es wird vermutet, daß durch Zerstörung des SSC1 Gens der Sortierungsmechanismus der Zelle beeinträchtigt wird, wodurch ein alternativer Sekretionsweg unter Umgehung des Golgi Kompartiments, in dem normalerweise die "outer chain" Glycosylierung stattfindet, eröffnet wird.

Die Nachteile der sscl-Mutation sind insbesondere:
* Die ssc1 Mutation verursacht ein Ca-abhängiges Wachstum. Die Mutantenstämme wachsen schlecht bei niedriger Ca-Konzentration.
* Die erhöhte Sekretion (z.B.: Prochymosin, u-PA und t-PA) ist abhängig vom Genprodukt. Die heterologe Sekretion von α-1-Antitrypsin und die Sekretion homologer Enzyme (Invertase ins Periplasma und alkalische Phosphatase, Proteinase B und Carboxy peptidase Y in die Vakuole) ist nicht erhöht (Moir, D.T., In: Barr, P.J.; Brake, A.J. et al., Yeast genetic engineering, Butterworths, Stoneham, Mass, pp. 215-231 (1989).

In Nagasu et al., Yeast 8 (1992) 535 - 547 und Nakayama et al., EMBO Journal 11 (1992) 2511 - 2519 wird das Hefegen OCH1 (im Weiteren auch als NGD29 bezeichnet) beschrieben. Nagasu und Nakayama beschreiben Hefemutanten (och1), welche eine Mutation im OCH1-Gen besitzen. Mit diesen Hefemutanten kann zwar eine Verringerung der outer chain Glycosylierung mit "^{H}-Mannose, Oligosaccharide (>Man₅₀GlcNAc₂)" bei der non-permissive-Temperatur erreicht werden. Mit diesen Hefemutanten ist es jedoch nicht möglich, daß in ihnen Proteine, insbesondere rekombinante Proteine, hergestellt werden können, die keine outer chain Glycosylierung des ^{H}-Mannose-Typs zeigen.

Huffaker et al. beschreibt in J. Biol. Chem. 257 (1982) 3203 - 3210 Hefe-mutanten im ALG-Gen, die mit alg1-Mutanten bezeichnet werden. Soche Mutanten zeigen jedoch bei der non-permissive-Temperatur von 37°C noch eine Glycosylierung, die zu 60 % dem Wildtyp (also eine Hyperglycosylierung mit umfangreicher outer chain Glycosylierung) umfaßt. Die von Huffaker beschriebenen Hefemutanten sind demnach nicht in der Lage, Proteine zu synthetisieren, die keine outer chain Glycosylierung tragen.

Die Aufgabe der vorliegenden Erfindung war es, diese Nachteile zu vermeiden und gut wachsende Hefewirtsstämme für die homologe und heterologe Sekretion von Proteinen zur Verfügung zu stellen mit möglichst einheitlicher und reduzierter N-Glycosylierung (z.B. mit einem vollständigen oder partiellen Defekt in der "outer chain" Glycosylierung), da in der Biotechnologie ein Bedarf besteht an gut wachsenden, gut sezernierenden Hefestämmen für die homologe und heterologe Sekretion von Proteinen mit einheitlicher definierter N-Glycosylierung.

Diese Aufgabe wird gelöst durch Hefemutanten mit Defekten in der N-Glycosylierung (ngd-Mutanten, "N-glycosylation defective"), die erhältlich sind durch [³H]-Mannose Suizidselektion, Einführung eines oder mehrerer selektiver Marker (Auxotrophiebedürfnisse und/oder Resistenzen) und Auswahl derjenigen Stämme, welche nach Transformation mit dem Plasmid YEpL/GOD und Anzucht in Vollmedium mit 2% Hefeextrakt, 4% Bactopepton, Difco, 0,1 mol/l Phosphatpuffer pH 7,0, 1% Fructose und 6% Maltose nach 3 - 4 Tagen Inkubation unter Schütteln GOD in einer Menge von 10 mg/l oder mehr produzieren und allel sind zu Saccharomyces cerevisiae DSM 7042, DSM 7160, DSM 7338 und/oder DSM 7340.

Das Prinzip der [³H]-Mannose Suizidselektion besteht im wesentlichen aus:
- Mutagenese (eines Wildtypstamms, z.B. von X2180-1A; ATCC 26786)
- Inkubation mit [³H]-Mannose
- Anreicherung von hyperglycosylierungsdefekten Mutanten durch Lagerung der Zellen bei tiefen Temperaturen, vorzugsweise bei etwa -80°C bis die Überlebensrate der Zellen auf 2 - 3 Zehnerpotenzen des ursprünglichen Werts abfällt, vorzugsweise beträgt die Lagerungsdauer dazu zwei bis vier Monate.
- Selektion nach Mutanten mit reduzierter N-Glycosylierung anhand von homolog exprimierter Invertase
- Analyse durch Aktivitätsanfärbung und/oder Immunpräzipitation von sezernierter Invertase und Bestimmung des Molekulargewichts der Invertase vorzugsweise durch SDS-PAGE. Aus dem ermittelten Molekulargewicht läßt sich der Umfang der Glycosylierung ermitteln.

Die [³H]-Mannose Suizidselektion ist eine wirksame Methode zur Herstellung und Isolierung von Hefemutanten (Littlwood, B.S., In: Methods of Cell Biology, Prescott, D.M. (ed), Academic Press, New York, (1975) Vol. IX, pp. 273-285). Bei diesem Verfahren wird durch tritium-markierte Mannose, welche z.B. in Glycoproteine der Zelle eingebaut wird, bei einem Teil der Hefezellen der Zelltod verursacht. Bei überlebenden Zellen erfolgt beispielsweise eine Änderung des Kohlenhydrat-metabolismus und/oder der Glycoproteinsynthese (Pouyssegur, J., Proc. Natl. Acad. Sci. 77, 2698-2701 (1980); Hirschberg, C.B. et al., Mol. Cell. Biol. 1 (1981), 902-909; Huffaker, T.C. und Robbins, P.W., J. Biol. Chem. 257 (1982), 3202-3210). Um Mutanten zu erhalten, die einen Defekt in der "outer chain" Glycosylierung von Hefemannoproteinen enthalten, wurde die [³H]-Mannose-Suizidselektioh gewählt. Dabei wurde von der Annahme ausgegangen, daß glycosylierungsdefekte Mutanten weniger radioaktive Mannose als Wildtypzellen einbauen und deshalb eine erhöhte Resistenz gegenüber der Exposition von [³H]-Mannose zeigen. Um dabei Unspezifitäten, die auf dem Metabolismus von Mannose beruhen, zu vermeiden, wird vorzugsweise an der Position zwei mit Tritium derivatisierte Mannose verwendet.

Die selektierbaren Marker (Auxotrophiemarker und/oder Resistenzen) können durch Kreuzung der Hefestämme zu Diploiden (Isolierung der Zygoten durch Mikromanipulation) und ggf. anschließende Sporulation zu Haploiden (Tetradenanalyse) eingeführt werden. Geeignete selektierbare Marker sind beispielsweise die Auxotrophiemarker ura3, leu2, trp1, lys2, his3, his4 und ade2 oder Gene, die eine Resistenz z.B. gegen Kupfer (CUP1 Gen) oder G418 (Tn601(903) Aminoglycosid Phosphotransferase Gen) bewirken (Bitter, G.A. et al., Methods Enzymol. 153 (1987) 516-543).

Vorzugsweise enthalten die N-glycosylierungsdefekten Mutanten einen Defekt im NGD29 und/oder NGD62 Gen.

Der ngd-Phänotyp kann durch Aktivitätsanfärbung von Invertase mittels nativer PAGE-Gele mit Saccharose und 2,3,4-Trinitrophenyltetrazoliumchlorid als Substrat/ Glucosereagenz ermittelt werden.

Die ausreichende GOD-Produktion kann bestimmt werden durch Aktivitätsbestimmung der ins Medium sezernierten GOD nach Anzucht unter Standardbedingungen. Dazu wird der zu testende Stamm (GOD Transformante) vorzugsweise nach einer selektiven Vorkulturführung in Vollmedium 3 - 4 Tage unter Schütteln inkubiert. Dem Vollmedium werden vorzugsweise Hefeextrakt, Bactopepton, Fructose und Maltose bei neutralem pH-Wert zugesetzt.

Die Bestimmung der Glucoseoxidase erfolgt beispielsweise nach dem in den Beispielen unter "Allgemeine Methoden" beschriebenen Verfahren.

Erfindungsgemäße Mutanten (allele Mutanten) können durch einen Test ermittelt werden, bei dem analysiert wird, ob die zu prüfenden Mutanten in den gleichen Genen eine Mutation aufweisen wie die Hefestämme DSM 7042 oder DSM 7338 (ngd29) und DSM 7160 oder DSM 7340 (ngd62).

Dazu wird der zu prüfende Stamm jeweils mit einem Hefestamm der Gruppe DSM 7042/7338 und der Gruppe DSM 7160/7340 gekreuzt und die dabei erhaltenen diploiden Stämme analysiert.

Die zu prüfende Mutation (Stamm) ist mit den erfindungsgemäßen ngd-Mutanten (DSM 7042, DSM 7338, ngd29) und/oder (DSM 7160, DSM 7340, ngd62) allel, wenn sich die Mutationen in diploiden Zellen nicht kompensieren.

Die zu prüfende Mutation (Stamm) ist mit den erfindungsgemäßen ngd-Mutanten (DSM 7042, DSM 7338, ngd29) und/oder (DSM 7160, ngd62) nicht allel, wenn sich die Mutationen in der diploiden Zelle komplementieren und ein Wildtypphänotyp hinsichtlich N-Glycosylierung resultiert.

Bevorzugte erfindungsgemäß verwendete Hefestämme sind die Stämme DSM 7042, DSM 7160, DSM 7338 und/oder DSM 7340. Besonders bevorzugt werden DSM 7338 und/oder DSM 7340 verwendet.

Ein weiterer Gegenstand der Erfindung ist ein Verfahren zur Herstellung von Saccharomyces Mutanten mit Defekten in der N-Glycosylierung durch [³H]-Mannosesuizidselektion, Einführung eines oder mehrerer selektierbarer Marker (und/oder Resistenzgene) Auxotrophiebedürfnisse und Auswahl derjenigen Stämme, welche nach Transformation mit dem Plasmid YEpL/GOD und Fermentation in Vollmedium mit 2% Hefeextrakt, 4% Bactopepton, Difco, 0,1 mol/l Phosphatpuffer pH 7,0, 1% Fructose und 6% Maltose nach 3 - 4 Tagen Inkubation unter Schütteln GOD in einer Menge von größer 10 mg/l produzieren und allel sind zu Saccharomyces cerevisiae DSM 7042, DSM 7160, DSM 7338 und/oder DSM 7340.

Ein weiterer Gegenstand der Erfindung ist die Verwendung der erfindungsgemäßen Hefestämme zur Herstellung von im wesentlichen einheitlich glycosylierten Proteinen. Zur Herstellung von hefeeigenen Glycoproteinen (z.B. externe Invertase, saure Phosphatase, Endoglucanase und Zellwand Mannoproteine) werden die erfindungsgemäßen Hefestämme fermentiert und das gewünschte Glycoprotein aus den Zellen oder dem Kulturüberstand nach bekannten Verfahren isoliert und gereinigt.

Heterologe Proteine werden dadurch erhalten, daß erfindungsgemäße Hefestämme mit einer rekombinanten DNA transformiert werden, welche das Gen des zu exprimierenden Glycoproteins enthält. Auf diese Weise können beispielsweise Glucoseoxidase, α1-Mikroglobulin, Erythropoietin und Glucoamylase hergestellt werden.

Ein weiterer Gegenstand der Erfindung ist ein Verfahren zur Expression und Herstellung von im wesentlichen einheitlich glycosylierten Proteinen durch Transformation einer Hefe-Mutante nach Anspruch 1 mit einer für das Protein codierenden DNA, Fermentation der Transformanten und Isolierung des Proteins aus den Zellen oder dem Kulturüberstand.

Für Patentzwecke wurden bei der Deutschen Sammlung für Mikroorganismen (DSM), Mascheroder Weg 1B,
D-3300 Braunschweig, hinterlegt:

| | | | Hinterlegungsnummer | Hinterlegungsdatum |
|---|---|---|---|---|
| 1. | Plasmid YEpL | | DSM 7038 | 07.04.1992 |
| 2. | Hefemutante | BMY3-9A (ngd29) | DSM 7042 | 08.04.1992 |
| 3. | " | BMY3-9C (ngd29) | DSM 7193 | 24.07.1992 |
| 4. | " | BMY12-20D (ngd62) | DSM 7160 | 09.07.1992 |
| 5. | " | BMY8-12A (ngd62) | DSM 7157 | 09.07.1992 |
| 6. | " | BMY13-7B (mnn9) | DSM 7158 | 09.07.1992 |
| 7. | " | BMY13-1C (mnn9) | DSM 7159 | 09.07.1992 |
| 8. | " | JM 1935 | DSM 7156 | 09.07.1992 |
| 9. | " | DBY 746 | DSM 4316 | 14.12.1987 |
| 10. | " | N-BMY3-9A | DSM 7338 | 08.12.1992 |
| 11. | " | N-BMY13-1C | DSM 7339 | 08.12.1992 |
| 12. | " | N-BMY12-20D | DSM 7340 | 08.12.1992 |
| 13. | " | N-BMY3-9C | DSM 7341 | 08.12.1992 |

Die nachfolgenden Beispiele erläutern die Erfindung weiter.

### Beispiele

### Allgemeine Methoden

### Rekombinante DNA-Technik

Zur Manipulation von DNA wurden Standardmethoden benutzt, wie sie bei Maniatis, T. et al., In: Molecular cloning: A laboratory manual. Cold Spring Harbor Laboratory Press, Cold Spring Harbor, New York, (1989), beschrieben sind. Die verwendeten molekularbiologischen Reagenzien wurden nach den Angaben des Herstellers eingesetzt.

### Hefetransformation

Saccharomyces cerevisiae Stämme wurden entsprechend der Methode von Beggs, J.D. (Nature 275 (1978) 104-109; Ito, H. et al., J. Bacteriol. 153 (1983) 163-168 oder Delorme, E. (Applied and Environmental Microbiology 55 (1989) 2242-2246) transformiert. Als C-Quelle für Glucoseoxidase exprimierende Hefestämme wurde Fructose anstelle von Glucose verwendet.

### Bestimmung der Glucoseoxidase Aktivität

Die GOD Aktivitätsbestimmung wurde in einem Volumen von 1 ml in mit Sauerstoff gesättigtem 0,1 mol/l Kaliumphosphatpuffer, pH 7,0, mit 0,18 mol/1 Glucose, 15 Units/ml Meerrettich Peroxidase und 1,75 mmol/l ABTS® Glucosereagenz bei 25°C durchgeführt. Die Reaktion wurde durch Zugabe von Glucoseoxidase (10 *µ*l GOD-haltige Probe verdünnt auf 5 - 20 mU/ml) gestartet und die Absorptionsänderung/min (ΔA/min) bei 405 nm (∈₄₀₅ = 36,8 [mmol⁻¹ x 1 x cm⁻¹]) bestimmt. 1 Unit (U) GOD Aktivität ist definiert als die Menge Enzym, die 1 *µ*mol Glucose pro min bei 25°C oxidiert. Die spezifische Aktivität der gereinigten A. niger GOD beträgt unter diesen Testbedingungen ca. 230 U/mg Protein.

### Proteinbestimmungen

Die Proteinbestimmung wurde nach der Microbiuret-Methode (Zamenhof, S. et al., Methods Enzymol. 3 (1957) 696-704) mit Rinderserumalbumin als Standard durchgeführt.

Die Proteinkonzentration gereinigter GOD Enzyme wurde anhand der optischen Dichte bei 280 nm ermittelt (1 OD₂₈₀ ≙ 1,5 mg/ml gereinigter GOD)

### Zell-Lyse und Rohextraktgewinnung

Die Zellen aus 5 ml Anzuchtmedium (ca. 0,1 - 0,2 g Hefe, Naßgewicht) wurden abzentrifugiert. Das Zellpellet wurde einmal mit 10 mmol/l Phosphatpuffer, pH 7,0, gewaschen und anschließend mit Glasperlen durch Homogenisieren mit einem Whirlmix aufgeschloseen (Ciriacy, M., Mut. Res. 29 (1975) 315-326). Danach wurden die Zellen in 2 ml 10 mmol/l Phosphatpuffer, pH 7,0, resuspendiert/extrahiert, die Zelltrümmer abzentrifugiert und der Überstand als Rohextrakt weiterverarbeitet.

### SDS-Polyacrylamid-Gelelektrophorese (SDS-PAGE)

Lösliche Proben (Mediumüberstände und Zell-Lysate) wurden mit 1/5 Volumen 5xSDS-Probenpuffer (1xSDS-Probenpuffer: 50 mmol/l Tris-HCl, pH 6,8, 1% SDS, 1% Mercaptoethanol, 10% Glycerin, 0,001% Bromphenolblau) versetzt und 5 min bei 95°C inkubiert. Nicht lösliche Proteine der Zelltrümmerfraktion wurden mit 2 ml 1xSDS-Probenpuffer und 6 - 8 mol/l Harnstoff extrahiert, durch 5 minütiges Erhitzen auf 95°C denaturiert und durch Zentrifugation von den unlöslichen Bestandteilen abgetrennt. Danach wurden die Proteine durch SDS-PAGE aufgetrennt (Laemmli, U.K., Nature 227 (1970) 680-685) und mit Coomassie Brilliant Blue® Farbstoff angefärbt.

### Beispiel 1

### Konstruktion von Plasmiden zur Sekretion der A. niger GOD in S. cerevisiae

### Konstruktion des Hefeexpressionsvektors YEpL (Basisvektor)

Das Plasmid YEpL basiert auf dem αGlucosidase Vektor YEp/5C6b3 (Kopetzki et al., Yeast 5 (1989) 11-24; Kopetzki, et al., EP-A 0 323 838). Zur Replikation des Plasmids in E. coli dient das ca. 2,3 kBp lange EcoRI/PvuII-Fragment aus dem Plasmid pBR322 (Plasmidursprung, Ampicillinresistenzgen). Zur Replikation in Hefe enthält der Vektor das ca. 2,2 kBp lange EcoRI-Fragment aus der 2*µ*m DNA der Befe (subkloniert aus dem E. coli/Hefe-Shuttlevektor YEp24). Weiterhin enthält der Vektor das URA3 und LEU2d Gen zur Selektion des Plasmides in auxotrophen Hefestämmen und eine αGlucosidase-Expressionskassette (GLUCPI-Gen). Sie besteht aus dem αGlucosidasepromotor, einem Polylinker (Klonierungstelle für zu exprimierende Gene) und dem αGlucosidaseterminator. Zudem ist das MAL2-8cp Gen vorhanden, dessen Genprodukt, das MAL2-8cp-Protein, den αGlucosidasepromotor aktiviert. Der αGlucosidasepromotor ist in Gegenwart von Glucose reprimiert. Er dereprimiert nach Verbrauch der Glucose und erreicht seine maximale Aktivität nach Induktion mit Maltose.

### 1.1 Konstruktion des Plasmids YEp/KL6b3

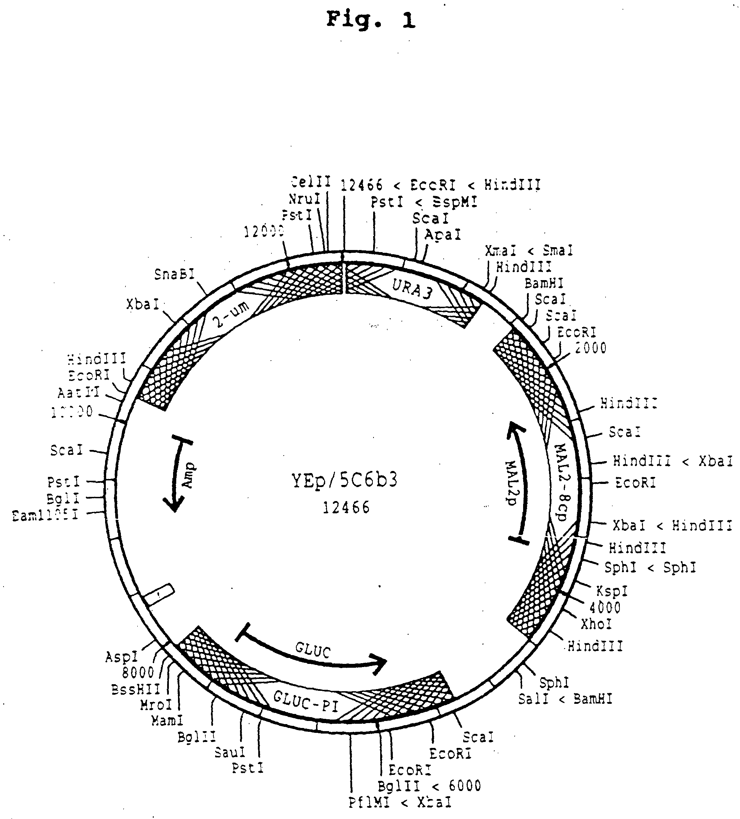

Die nicht benötigte ca. 1,4 kBp lange DNA Sequenz zwischen dem αGlucosidaseterminator und dem MAL2-8cp Promotor wurde aus dem Plasmid YEp/5C6b3 (Fig. 1) deletiert.

Dazu wurde das Plasmid YEp/5C6b3 mit XhoI linearisiert, die 5'-überhängenden Enden mit Klenow Polymerase aufgefüllt, das Plasmid mit MroI nachgeschnitten und das 8,7 kBp lange MroI/XhoI(blunt)-Vektorfragment isoliert. In einem zweiten Ansatz wurde das Plasmid YEp/5C6b3 mit den Restriktionsendonukleasen MroI und ScaI verdaut, das αGlucosidasegen enthaltende 2,5 kBp lange MroI/ScaI-Fragment isoliert und mit dem 8,7 kBp langen MroI/XhoI(blunt)-Vektorfragment ligiert. Das gewünschte Plasmid wurde durch Restriktionskartierung identifiziert und mit YEp/KL-6b3 bezeichnet.

### 1.2 Konstruktion des Plasmids YEp/KL-6b3M

In die SspI Restriktionsendonukleaseschnittstelle der 5'-nichttranslatierten Region des MAL2-8cp Gens wurde ein MluI-Linker (5'-GACGCGTC-3') ligiert. Plasmidkonstruktion: YEp/KL-6b3M.

### 1.3 Konstruktion des Plasmids YEp/KL-6b3M-MCS

Durch "polymerase chain reaktion" (PCR)-Technik (Mullis, K.B. und Faloona, F.A., Methods in Enzymol. 155 (1987) 335-350) wurde das Strukturgen der αGlucosidase entfernt und durch einen DNA-Linker ("multi cloning site", MCS) ersetzt.

Dazu wurde die GLUCPI-Promotorsequenz aus dem Plasmid YEp/KL-6b3M mittels PCR unter Verwendung des Primerpaares (siehe SEQ ID NO. 1 und SEQ ID NO. 2) amplifiziert und das ca. 410 Bp lange PCR Produkt nach Agarosegelelektrophorese isoliert.

In einer zweiten PCR Reaktion wurde die GLUCPI-Terminatorsequenz aus dem Plasmid YEp/KL-6b3M unter Verwendung des Primerpaares (siehe SEQ ID NO. 3 und SEQ ID NO. 4) amplifiziert und das ca. 860 Bp lange PCR Produkt nach Agarosegelelektrophorese isoliert.

Danach wurden äquimolare Mengen (jeweils ca. 50 pg) der isolierten PCR-Fragmente in PCR Reaktionsmix vereinigt, 5 min bei 95°C zur Denaturierung der ds-DNA inkubiert, das Reaktionsgemisch auf 60°C zum "Annealing" der komplementären MCS-haltigen singulären DNA Stränge abgekühlt, die Hybridisierungsprodukte mit Taq-Polymerase in ds-DNA überführt und in einer dritten PCR Reaktion unter Verwendung des Primerpaares (siehe SEQ ID NO. 1 und SEQ ID NO. 4) amplifiziert. Danach wurde das ca. 1,27 kBp lange PCR Produkt mit den Restriktionsendonukleasen MroI und MluI verdaut, das ca. 0,92 kBp lange MroI/MluI-GLUPI-Promotor/MCS/GLUCPI-Terminatorfragment nach Agarosegelelektrophorese isoliert und in das ca. 8,55 kBp lange MroI/MluI-YEp/KL-6b3M Vektorfragment ligiert. Das gewünschte Plasmid YEp/KL-6b3M-MCS wurde mittels Restriktionskartierung identifiziert und die mittels PCR synthetisierten DNA-Bereiche durch DNA Sequenzierung überprüft.

### 1.4 Konstruktion des Plasmids YEpL

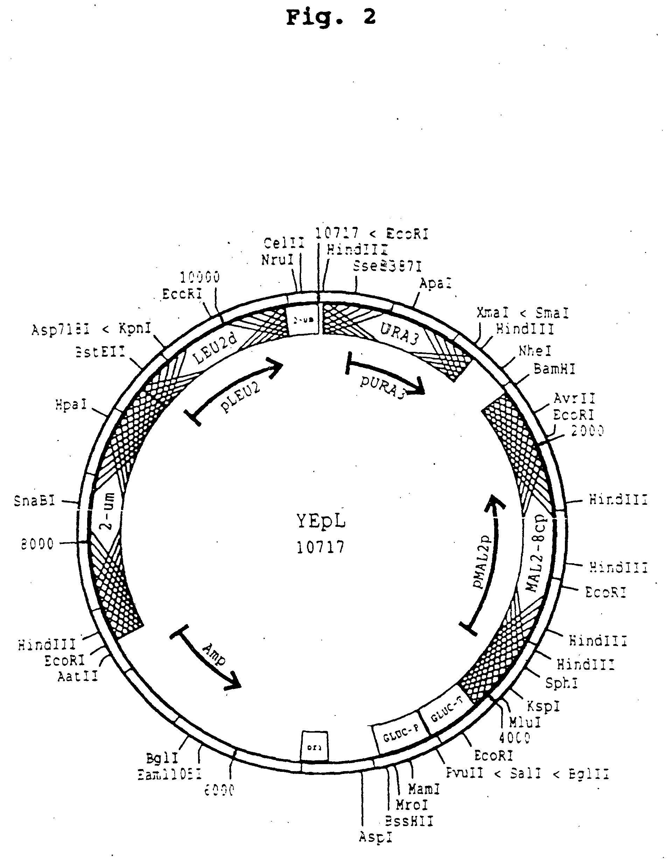

In der folgenden Plasmidkonstruktion wurde das LEU2d Gen in das Plasmid YEp/KL-6b3M-MCS insertiert. Dazu wurde das Plasmid YEp/KL-6B3M-MCS mit CelII und SnaBI verdaut und das 8,4 kBp lange CelII/SnaBI-YEp/KL-6b3M-MCS Vektorfragment isoliert. Das LEU2d Gen wurde als ca. 2,32 kBp langes CelII/SnaBI-Fragment aus dem Plasmid pADH040-2 (Erhart, E. und Hollenberg, C.P., J. Bacteriol. 156 (1983) 625-635) isoliert und mit dem 8,4 kBp langen CelII/SnaBI-YEp/KL-6b3M-MCS Vektorfragment ligiert. Die gewünschte Plasmidkonstruktion YEpL (DSM 7038) wurde durch Restriktionskartierung identifiziert (Fig. 2).

### 1.5 Konstruktion des Plasmids YEpL/GOD

Die Klonierung des verwendeten Glucoseoxidase Gens (Stamm: NRRL-3, ATTC 9029), die Subklonierung in den pBluescript SK(+) Vektor, die DNA Sequenzierung und die Ableitung der GOD Proteinsequenz sind in der Publikation von Kriechbaum, M. et al. (FEBS Lett. 255 (1989) 63-66) beschrieben. Das GOD Gen wurde in 2 Teilbereichen (SalI Restriktionsfragmente) in pBluescript SK(+) kloniert.

Das Plasmid pSK/GOD-1.8 enthält ein ca. 1,8 kBp langes SalI-Fragment, das für die 5'- nichttranslatierte Region und den N-terminalen Bereich des GOD Strukturgens bis zur SalI-Schnittstelle an Bp-Position 164 kodiert (Bp-Position entsprechend der Numerierung bei Kriechbaum, M. et al.). Das Plasmid pSK/GOD-2.0 enthält ein ca. 2,0 kBp langes SalI-Fragment, das für den Rest des GOD Strukturgens von Bp-Position 165 bis 1853 sowie die 3'- nichttranslatierte Region stromabwärts des GOD Strukturgens kodiert.

Mittels PCR Technik wurde die 5'- und 3'- nichttranslatierte Region des GOD Gens entfernt, das GOD Strukturgen an beiden Enden mit singulären Restriktionsendonukleaseschnittstellen versehen (BglII bzw. PvuII) und zudem in der C-terminalen kodierenden Region des GOD Strukturgens eine singuläre SphI und NheI Schnittstelle eingeführt unter Erhalt einer für das native GOD Protein kodierenden DNA-Sequenz. Danach wurde das GOD Strukturgen in einer Dreifragmentligation aus den beiden PCR Fragmenten zusammengesetzt und in den Vektor YEpL inseriert. Zur Amplifikation des N-terminalen GOD Strukturgens wurde das folgende Primerpaar verwendet (siehe SEQ ID NO. 5 und SEQ ID NO. 6) und Plasmid pSK/GOD-1.8 als Template DNA.

Zur Amplifikation des restlichen GOD Strukturgens wurde das folgende Primerpaar verwendet (siehe SEQ ID NO. 7 und SEQ ID NO. 8) und Plasmid pSK/GOD-2.0 als Template DNA.

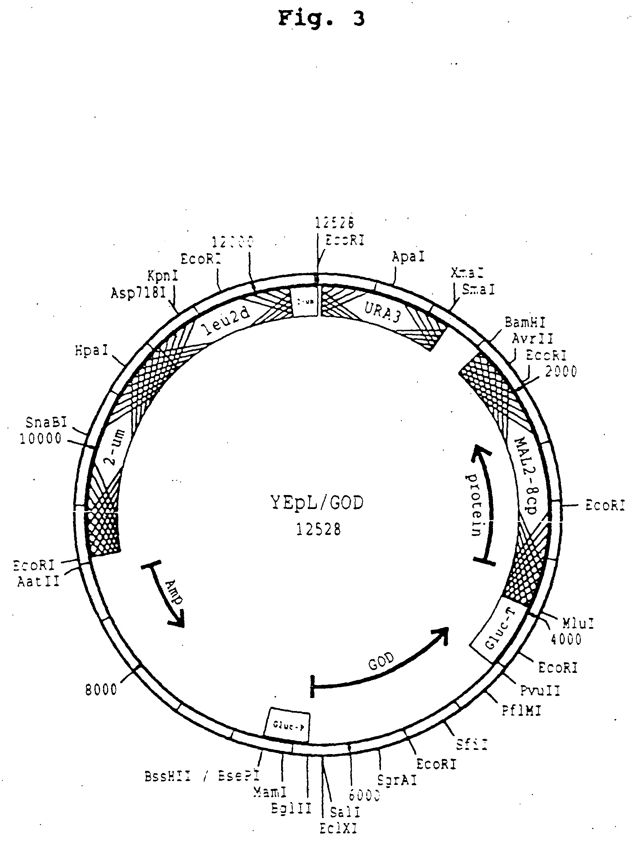

Das ca. 220 Bp lange PCR Produkt der ersten Reaktion wurde mit BglII und SalI nachgespalten und das ca. 130 Bp lange BglII/SalI-Fragment isoliert. Das ca. 2,05 kBp lange PCR Produkt der zweiten Reaktion wurde mit SalI und PvuII verdaut das ca. 1,7 kBp lange DNA Fragment isoliert. Danach wurden die PCR Fragmente in das ca. 10,7 kBp lange BglII/PvuII-YEpL Vektorfragment ligiert (Dreifragmentligation). Das gewünschte Plasmid YEpL/GOD (Fig. 3) wurde durch Restriktionskartierung identifiziert und partiell sequenziert (Klonierungsübergänge).

### 1.6 Konstruktion des Plasmids YEpL/GOD-(His)₄

Das Plasmid enthält ein modifiziertes GOD Gen, das für eine GOD Enzymvariante kodiert, die C-terminal zusätzlich 4 Histidinreste besitzt. YEpL/GOD-(His)4 wurde aus dem Plasmid YEpL/GOD hergestellt.

Dazu wurde das Plasmid YEpL/GOD partiell mit SphI und vollständig mit PvuII gespalten, das ca. 10,7 kBp lange SphI/PvuII-Fragment isoliert und mit dem folgenden, aus 2 Oligonukleotiden (siehe SEQ ID NO. 9 und SEQ ID NO. 10) durch Hybridisierung hergestellten, DNA Linker ligiert.

Das gewünschte Plasmid YEpL/GOD-(His)₄ wurde durch Koloniehybridisierung mit radioaktiv markiertem Primer 10 als Sonde idendifiziert und durch Restriktionskartierung und partielle Sequenzierung (C-terminale Bereich des GOD Strukturgens) weiter analysiert.

### Beispiel 2

### Isolierung von Hefewirtsstämmen mit defekter N-Glycosylierung

### 2.1 [³H]-Mannose Suizidmutagenese

### Prinzip:

- Mutagenese (Ausgangsstamm: X2180-1A, Genotyp: a SUC2 mal mel gal2 CUP1; ATCC 26786)
- Inkubation mit [³H]-Mannose
- Anreicherung von hyperglycosylierungsdefekten Mutanten durch Lagerung der Zellen bei -80°C bis die Überlebensrate der Zellen auf 2 - 3 Zehnerpotenzen abfällt (2 - 4 Monate)

Ein Hefestamm wie z. B. X2180-1A (ATCC 26786) wird in YEPD-Medium (2% Bacto Pepton, 1% Hefeextrakt, Difco, und 4% Glucose) angezogen, in der logarithmischen Wachstumsphase (davon ca. 5 x 10⁸ Zellen) geerntet, mit 0,1 mol/l Natriumphosphat, pH 7, gewaschen und in 1,5 ml 0,1 mol/l Natriumphosphat, pH 7, resuspendiert. Die Zellen werden mutagenisiert durch Zusatz von 0,1 ml Ethylmethansulfonat für eine Stunde bei 25°C. 0,2 ml der so behandelten Zellen werden mit 10 ml Natriumthiosulfat (5% w/v) für 10 Minuten inkubiert, 3 x mit 0,1 mol/l Natriumphosphat, pH 7,0, gewaschen und in YEPD-Medium (2% Bacto Pepton, 1% Hefeextrakt, Difco und 4% Glucose) resuspendiert.

Die Zellen werden bei 28°C unter Schütteln inkubiert bis eine OD von 0,6 bei 578 nm erreicht ist. 10⁶ Zellen werden mit YEP-Medium (2% Bacto Pepton, 1% Hefeextrakt, Difco) gewaschen und in 0,1 ml YEP mit 0,1% Glucose resuspendiert. 2 mCi [³H]-Mannose (spezifische Aktivität 18,5 Ci/mmol) werden zugegeben und die Kultur bei 28°C für 60 Minuten inkubiert. Die Zellen werden abzentrifugiert, mit Wasser gewaschen und in YEPD, welches 25% Glyzerin enthält, resuspendiert und bei -70°C zur Einwirkung der Radioaktivität gelagert. Nach ca. 45 - 50 Tagen, wenn die Überlebensrate der Zellen auf 1,5 - 0,2% abgefallen ist, werden Aliquote der Zellen auf YEP Agarplatten mit 2% Mannose ausplattiert und bei 30°C inkubiert.

### 2.2 Isolierung von Mutanten mit reduzierter N-Glycosylierung

Mutanten mit einem Defekt in der Proteinglycosylierung werden zunächst auf ihre Fähigkeit selektioniert [³H]-Mannose nicht einzubauen und [³⁵S]-Methionin einzubauen. Dazu läßt man die Zellen auf YEPD-Agar-Platten wachsen, repliziert die Hefekolonien auf 2 Rotband Filter (Schleicher & Schüll, Dassel, Deutschland) und inkubiert die Filter nochmals auf YEPD-Platten für 6 Stunden. Ein Filter wird dann in einer Lösung aus YEPD (eine zur Benetzung des Filters gerade ausreichende Menge), welche 0,01 mCi/ml [³⁵S]-Methionin enthält, inkubiert. Der andere Filter wird mit YEP, welches 0,2 mCi/ml [³H]-Mannose enthält, getränkt und 30 Minuten inkubiert. Die Zellen/Kolonien werden mit 5% Trichloressigsäure auf dem Filter fixiert, mit Wasser und Aceton gewaschen und durch Autoradiographie analysiert.

### 2.3 Charakterisierung der positiven Klone durch native Gelelektrophorese von externer Invertase

Das SUC2 Gen aus S. cerevisiae kodiert für 2 unterschiedlich regulierte und kompartimentierte Invertase Formen, i) eine glycosylierte vorwiegend ins Periplasma sezernierte Invertase und ii) eine intrazelluläre etwas verkürzte nicht glycosylierte Form (Carlson, M. et al., Mol. Cell. Biol. 3 (1983) 439-447). Die Invertase enthält 14 potentielle N-Glycosylierungsstellen, von denen im Durchschnitt 9 - 10 bei der sezernierten Form pro Invertase Untereinheit glycosyliert werden. Externe Wildtyp Invertase wandert durch nicht einheitliche "outer chain" Glycosylierung als diffuse Bande in nativen Gelen. Die cytoplasmatische, nicht glycosylierte Form ergibt im Gegensatz dazu eine scharfe Bande nach Aktivitätsanfärbung. Eine veränderte N-Glycosylierung läßt sich somit über die Wanderungsgeschwindigkeit und die Bandenschärfe von externer, Invertase in nativen Gelen grob analysieren.

Die Hefestämme (X2180-1A Wildtypstamm und positive Klone) wurden in 5 ml YEPS-Medium (1% Hefeextrakt, 2% Bactopepton, Difco, und 2% Saccharose) über Nacht angezogen, die Zellen in der spätlogarithmischen Wachstumsphase geerntet, einmal mit 20 mmol/l Natriumazid gewaschen und mit Glasperlen durch Homogenisieren auf einem Whirlmix aufgeschlossen. Die Zelllysat-Herstellung, die native Gelelektrophorese und Aktivitätsanfärbung von Invertase mit Saccharose und 2,3,4-Trinitrophenyltetrazoliumchlorid als Substrat/Glucosereagenz wurde entsprechend der Methode von Ballou C.E. (Methods Enzymol. 185 (1990) 440-470) durchgeführt.

Die positiven Klone lassen sich anhand der Invertase Aktivitätsanfärbung in 4 Klassen einteilen:
1. Mutanten mit Wildtyp Invertase Mobilität.
2. Mutanten, die weder nichtglycosylierte noch glycosylierte Invertase synthetisieren.
3. Mutanten mit Defekten in der "outer chain" Glycosylierung (distinktes oligomeres Bandenmuster aus 3 - 4 Banden).
4. Mutanten, die zu einer ausgeprägten Unterglycosylierung von Invertase führen (größere Mcbilität als Wildtyp Invertase)

### Ergebnis

Mutantenstämme der Klasse 4, im weiteren mit ngd29 (DSM 7042/7338) und ngd62 (DSM 7160/7340) bezeichnet (ngd für: "N-glycosylation defective"), synthetisieren im Vergleich zum Ausgangsstamm X2180-1A eine "einheitlich glycosylierte" dimere externe Invertase (scharfe Bande und erhöhte Wanderungsgeschwindigkeit in nativen Gelen nach Aktivitätsanfärbung). Die ngd-Mutantenstämme waren osmotisch stabil, bei 30°C kultivierbar und aggregierten nicht während der Anzucht.

### Beispiel 3

### Konstruktion von glycosylierungsdefekten Hefewirtsstämmen zur Expression homologer und heterologer Proteine

Zur Einführung eines oder mehrerer durch Transformation komplementierbarer Auxotrophien wurden die ngd-Mutanten mit geeigneten Laborstämmen entsprechend der bei F. Sherman et al. (Methods in Yeast Genetics: A Laboratory Manual, Cold Spring Harbor Laboratory, Cold Spring Harbor, New York, (1981)) beschriebenen Methode gekreuzt und diploide Stämme durch Mikromanipulation isoliert. Anschließend wurden die diploiden Stämme sporuliert und Segreganten mit geeigneten Auxotrophien (z.B. ura3, leu2) und ngd-Mutation isoliert.

Dazu wurde die ngd29-Mutante zusammen mit dem Stamm DBY746 (MATα ura3-52 leu2-3,-112 trp1-289a his3-Δ1; [DSM 4316] äquivalent mit ATCC 44733) und die ngd62 Mutante mit dem Stamm JM1935 (MATα ura3 leu2 his4, DSM 7156) 6 Stunden bei 30°C in YEPD (1% Hefeextrakt, 2% Bactopepton, Difco, und 2% Glucose) inkubiert. Anschließend wurden mit Hilfe eines Mikromanipulators (Modell: nach de Fonbrune der Firma Bachhofer, Reutlingen) Zygoten isoliert und in 5 ml YEPD über Nacht angezogen. Die Zellen wurden kurz abzentrifugiert, das Medium bis auf ca. 0,2 ml dekantiert und das Zellpellet im Restmedium resuspendiert. Diese Zellsuspension wurde auf eine Kaliumacetat-Platte (1% Kaliumacetat, 1,5% Agar) ausgebracht. Nach ca. 5 Tagen wurden die so erhaltenen Asci mit einer Impföse in 0,5 ml sterilem Wasser resuspendiert, 10 µl eines β-Glucuronidase/Arylsulfatase-Gemisches (Boehringer Mannheim) zugegeben und 10 min bei Zimmertemperatur inkubiert. Anschließend wurden 10 ml Wasser zugegeben, die Ascus-Suspension abzentrifugiert und der Überstand dekantiert. Unter dem Mikromanipulator wurden die Sporen mehrerer Asci isoliert und auf YEPD-Platten (YEPD mit 1,5% Agar) 3 Tage bei 30°C inkubiert. Von ausgekeimten Sporen wurde eine Stempelplatte angelegt, die Kolonien auf synthetischen Minimalmedien (0,67% Yeast Nitrogen Base ohne Aminosäuren, Difco; 2% Glucose; 1,5% Agar plus Zusätze: 20 mg/l Trp, His, Arg, Met; 30 mg/l Leu, Ile, Lys; 50 mg/l Phe; 100 mg/l Glu, Asp; 400 mg/l Val, Thr, Ser sowie 20 mg/l Adenin und Uracil; jeweils einer dieser Zusätze fehlte in den einzelnen Minimalmedien) gestempelt und 3 Tage bei 30°C inkubiert. Segreganten mit ngd29-Phänotyp, die Auxotrophien für Uracil und Leucin aufwiesen, wurden wie in Beispiel 2.2. beschrieben analysiert/isoliert. In allen untersuchten Tetraden segregierte der ngd29-Phänotyp (ebenso wie der ngd62 Phänotyp) 2:2, was für eine einzelne Mutation in einem einzelnen nuklären Locus spricht.

Aus der hier beschriebenen Kreuzung DBY746 x ngd29 wurden die Stämme BMY3-9A und N-BMY3-9A (MATα leu2-3,112 ura3-52 his3-Δ1 ngd29; DSM 7042 und DSM 7338) und BMY3-9C und N-BMY3-9C (MATα leu2-3, 112, ura3-52 ngd29; DSM 7193 und DSM 7341) erhalten.

Auf analoge Art und Weise wurde aus der Kreuzung JM1935 x ngd62 die Stämme BMY12-20D und N-BMY12-20D (MATα leu 2 ura 3 his 4 ngd62; DSM 7160 und DSM 7340) erhalten.

### Beispiel 4

### Vergleich der Expression/Sekretion von nativer A. niger GOD und der GOD-(His)₄ Variante in Wildtyp und glycosylierungsdefekten Hefewirtsstämmen

Die GOD aus A. niger ist ein natürlicherweise sezerniertes, glycosyliertes dimeres Enzym. Pro Untereineinheit sind 8 potentielle N-Gycosylierungsstellen (Sequons) und 3 Cysteinreste vorhanden, von denen zwei eine Disulfidbrücke ausbilden. In S. cerevisiae Wildtypstämmen exprimierte GOD wird ins Medium sezerniert und ist hinsichtlich des Molekulargewichts auf Grund einer nicht einheitlichen "outer chain" Glycosylierung (Hyperglycosylierung) sehr heterogen (Frederick, K.R. et al., J. Biol. Chem. 265 (1990) 3793-3802; De Baetselier, A. et al., Biotechnology 9 (1991) 559-561; Whittington, H. et al., Curr. Genet. 18 (1990) 531-536). Das prozessierte (Abspaltung einer 22 Aminosäuren langen Signalsequenz) A. niger GOD Protein besteht aus 583 Aminosäuren mit einem potentiellen Molekulargewicht von 63 273 Da (Frederick, K.R. et al., J. Biol. Chem. 265 (1990) 3793-3802).

Die Plasmide YEpL/GOD (Beispiel 1.5) und YEp/GOD-(His)₄ (Beispiel 1.6) wurden in den Wildtypstamm JM1935 (MATα leu2 ura3 his4 MAL4) DSM 7156 BMY3-9A (DSM 7042 und N-BMY3-9A (DSM 7338) (siehe Beispiel 3) transformiert und die Transformanten auf Minimalmediumagarplatten mit 1,5% Agarose, 0,67% YNB (Yeast Nitrogen Base, Salz-Vitamin-Gemisch, Difco) 0,5 % CAA (Casaminosäuren, Proteinhydrolysat, Difco) und 2% Fructose als C-Quelle selektioniert (Uracil-Selektion).

### 4.1 Anzucht der GOD-Transformanten

Zur Amplifikation der Plasmidkopienzahl (Selektion auf das Plasmid-kodierte LEU2d-Allel; Beggs, J.D., Nature 275 (1978) 104-109; Erhart, E. und Hollenberg, C.P. J., Bacteriol. 156 (1983) 625-635) wurden die Transformanten auf Minimalmediumplatten ohne Leucin ausgestrichen (1,5% Agarose, 0,67% YNB , Difco, 60 mg/l Adenin und 2% Fructose).

Vorkulturanzuchten wurden in Leucin Selektivmedium mit 0,67% YNB und 4% Fructose in Schüttelkolben bei 30°C für 48 Std. durchgeführt und zum Animpfen von Expressionskulturen (Inoculum: 1 - 2%) verwendet. Die Hauptkultur (1 l Schüttelkultur) wurde bei 30°C in Vollmedium mit 2% Hefeextract, 4% Bactopepton, Difco, 0,1 mol/l Phosphatpuffer, pH 7,0, 1% Fructose und 6% Maltose für 3 - 4 Tage unter Schütteln inkubiert. Nach 48 und 72 Std. wurden Proben entnommen und das Zellwachstum (Bestimmung der optischen Dichte bei 600 nm, OD₆₀₀), die ins Medium sekretierte GOD Aktivität und die in den Zellen verbleibende GOD Aktivität nach Zell-Lyse im Rohextrakt bestimmt.

### Expressions-/Sekretionsanalyse von GOD in dem Wildtypstamm DSM 7156 und den glycosylierungsdefekten Wirtsstämmen DSM 7042/7338 (ngd29)

| **Plasmid: YEpL/GOD** | | | | |
|---|---|---|---|---|
| | GOD-Aktivität (U/ml) / Optische Dichte (OD₆₀₀) | | | |
| DSM 7156 | Zeit (Std.) | | | |
| | 48 U/ml / OD₆₀₀ | | 72 U/ml / OD₆₀₀ | |
| extrazellulär | 8 | 13 | 12 | 17 |
| intrazellulär | 4 | | 6 | |
| gesamt | 12 | | 18 | |
| % sezerniert | 66 | | 66 | |

| **Plasmid: YEpL/GOD** | | | | |
|---|---|---|---|---|
| | GOD-Aktivität (U/ml) / Optische Dichte (OD₆₀₀) | | | |
| DSM 7042/7338 | Zeit (Std.) | | | |
| | 48 U/ml / OD₆₀₀ | | 72 U/ml / OD₆₀₀ | |
| extrazellulär | 11 | 9 | 18 | 14 |
| intrazellulär | 1 | | 2 | |
| gesamt | 12 | | 20 | |
| % sezerniert | 87 | | 90 | |

### Expressions-/Sekretionsanalyse von GOD-(His)₄ in dem Wildtypstamm DSM 7156 und den glycosylierungsdefekten Wirtsstämmen DSM 7042/7338 (ngd29)

| **Plasmid: YEpL/GOD-(His)**_{**4**} | | | | |
|---|---|---|---|---|
| | GOD-Aktivität (U/ml) / Optische Dichte (OD₆₀₀) | | | |
| DSM 7156 | Zeit (Std.) | | | |
| | 48 U/ml / OD₆₀₀ | | 72 U/ml / OD₆₀₀ | |
| extrazellulär | 8 | 14 | 9 | 14 |
| intrazellulär | 5 | | 6 | |
| gesamt | 13 | | 16 | |
| % sezerniert | 62 | | 58 | |

| **Plasmid: YEpL/GOD** | | | | |
|---|---|---|---|---|
| | GOD-Aktivität (U/ml) / Optische Dichte (OD₆₀₀) | | | |
| DSM 7042/7338 | Zeit (Std.) | | | |
| | 48 U/ml / OD₆₀₀ | | 72 U/ml / OD₆₀₀ | |
| extrazellulär | 12 | 10 | 17 | 13 |
| intrazellulär | 1 | | 1 | |
| gesamt | 13 | | 18 | |
| % sezerniert | 88 | | 93 | |

### Ergebnis

Hinsichtlich Expression und Sekretion wurden für die GOD und GOD-(His)₄ Variante keine signifikanten Unterschiede gefunden.

### 4.2 SDS-PAGE von sezernierter GOD

Das in den glycosylierungsdefekten Wirtsstämmen DSM 7042/7338 (ngd29) und DSM 7160/7340 (ngd62) exprimierte (ins Medium sezernierte) GOD-(His)₄ Enzym wurde mit dem in dem Wildtypstamm DSM 7156 exprimierten (sezernierten) Enzym und gereinigter GOD aus A. niger (Boehringer Mannheim, BRD) durch SDS-PAGE und anschließende Proteinanfärbung näher charakterisiert. Die GOD-haltigen Mediumüberstände aus dem Wildtypstamm wurden vor der Elektrophorese 10fach durch TCA-Fällung konzentriert. Kohlenhydratfreies GOD-(His)₄ Enzym wurde enzymatisch mit N-Glycosidase F hergestellt und als Größenstandard verwendet.

### Enzymatische Deglycosylierung mit N-Glycosidase F

Die Deglycosylierung wurde in Anlehnung an die von Haselbeck, A. und Hösel, W. publizierte Methode (Topics in Biochemistry 8 (1988) 1-4) durchgeführt. 0,1 ml GOD-(His)₄-haltiger Mediumüberstand wurde mit Trichloressigsäure (Endkonzentration: 10%) gefällt, die präzipitierten Proteine abzentrifugiert, das Proteinpellet mit 70% Ethanol gewaschen, im Vakuum getrocknet, in 10 *µ*l 20 mmol/l Kaliumphosphatpuffer, pH 7,2, mit 1% SDS aufgenommen und 3 min auf 95°C erhitzt. Nach Abkühlung auf Raumtemperatur wurde die Probe mit 20 mmol/l Kaliumphosphatpuffer, pH 7,2, Octylglucosid (Endkonzentration: 0,5%) und 5 Units N-Glycosidase F auf 0,1 ml verdünnt, 1 - 12 Std. bei 37°C inkubiert und anschließend mit 25 *µ*l 5xSDS-Puffer (siehe oben) versetzt.

### Ergebnis

Die in den glycosylierungsdefizienten ngd-Mutantenstämmen exprimierten GOD Enzyme (GOD und GOD-(His₄)) sind als dominante einheitliche Bande mit einem Molekulargewicht von ca. 80 kDa nach Proteinanfärbung in SDS-PAGE Gelen sichtbar. Dieses Experiment zeigt die fehlende "outer chain" Glycosylierung der GOD Enzyme und deutet auf eine einheitliche "core" ähnliche Glycosylierung hin. Die ngd-Mutantenstämme weisen hinsichtlich der Glycosylierung einen mnn9 ähnlichen Phänotyp auf. Im Gegensatz dazu sind die in Wildtypstämmen exprimierten GOD Enzyme nur als sehr diffuse Banden zu erkennen, die sich über einen Molekulargewichtsbereich von ca. 80 - 200 kDa erstrecken.

### Beispiel 5

### Charakterisierung der N-glycosylierunadefekten ngd-Mutanten anhand des Wachstums auf YEPD Agarplatten mit Orthovanadat oder Hygromycin B

Glycosylierungsdefekte Mutanten wie z.B. mnn8, mnn9 und mnn10 zeigen eine erhöhte Orthovanadat Resistenz und eine erhöhte Sensitivität gegenüber dem Antibiotikum Hygromycin B. Der Resistenz-/Sensitivitätsphänotyp ermöglicht eine Unterscheidung/Gruppierung von N-glycosylierungsdefekten Mutanten (Ballou, L. et al., Proc. Natl. Acad. Sci. 88 (1991) 3209-3212).

Die zu untersuchenden Stämme wurden in YEPD Medium (5 ml Rollerkultur) über Nacht angezogen und die Stämme/Kulturen mit YEPD Medium auf eine optische Dichte (OD₆₀₀) von exakt 0,05 eingestellt. Danach wurden jeweils 20 *µ*l Zellsuspension auf YEPD-Agarplatten mit 2 - 15 mmol/l Natriumorthovanadat oder 10 - 200 *µ*g/ml Hygromycin B gespottet. Nach 2 Tagen Inkubation bei 30°C wurde das Wachstum der Zellflecken ausgewertet (siehe Tabelle).

### Wachstumsphänotyp von Hefezellen auf YEPD Agarplatten mit Natriumorthovanadat oder Hygromycin B

| Stamm | Orthovanadat Resistenz mmol/l | | | | | | | | Hygromycin Resistenz (µg/ml) | | | |
|---|---|---|---|---|---|---|---|---|---|---|---|---|
| | 2 | 3 | 4 | 5 | 6 | 7 | 10 | 15 | 10 | 50 | 100 | 200 |
| DBY 746 (Wildtyp) | + | + | + | + | - | - | - | - | + | + | - | - |
| X2180-1A (Wildtyp) | + | + | + | + | - | - | - | - | + | + | - | - |
| LB347-1C (mnn9)¹⁾ | + | + | + | + | + | + | + | + | - | - | - | - |
| BMY3-9A (ngd29) | + | + | + | + | + | - | - | - | + | ± | - | - |
| BMY12-20D(ngd62) | + | + | + | ± | - | - | - | - | + | ± | - | - |
| N-BMY3-9A(ngd29) | + | + | + | + | + | - | - | - | + | ± | - | - |
| N-BMY12-20D(ngd62) | + | + | + | ± | - | - | - | - | + | ± | - | - |
| + Wachstum ± sehr langsames Wachstum - kein Wachstum | | | | | | | | | | | | |

| | | | | | | | | | | | | |
|---|---|---|---|---|---|---|---|---|---|---|---|---|
| ¹⁾ J. Biol. Chem. 259 (1984) 3805-3811 | | | | | | | | | | | | |

### Ergebnis

Die ngd-Mutanten zeigen Unterschiede hinsichtlich des Resistenzmusters untereinander, zur mnn9 Mutante und zu Wildtypstämmen auf.

### Beispiel 6

### Charakterisierung/Identifizierung der ngd-Mutanten (Allelietest)

Ein Allelietest dient zur Identifizierung (Unterscheidung) von Genen und Gendefekten (Mutationen). Mit dieser Technik läßt sich analysieren, ob 2 Mutanten allel sind (im gleichen Gen eine Mutation aufweisen). Die ngd-Mutanten wurden untereinander und zur mnn9 Mutante auf Allelie untersucht.

Die Allelieteste wurden mittels genetischer Standardtechniken durchgeführt (siehe: Sherman, F.; Fink, G.R.; Hicks, J.B., Methods in Yeast Genetics: A Laboratory Manual. Cold Spring Harbor Laboratory, Cold Spring Harbor, New York, (1981); Guthrie, C. und Fink, G.R. (eds.), Guide to Yeast Genetics and Molecular Biology. Methods Enzymol. 194 (1991)).

### Prinzip:

Zwei zu analysierende haploide Mutantenstämme unterschiedlichen Paarungstyps mit sich komplementierenden Auxotrophiebedürfnissen werden gekreuzt und die diploiden Stämme auf Platten mit Minimalmedium selektioniert. Die Diploidie der isolierten Stämme wird durch das Vorhandensein von a und α Paarungstyp spezifischen DNA Sequenzen mittels PCR Analyse entsprechend der Methode von Huxley, C. et al. (Trends Genet. 6 (1190) 236) bestätigt.

Zwei Mutanten sind allel, d.h. besitzen eine Mutation im gleichen Gen, wenn sich die Mutationen in der diploiden Zelle nicht komplementieren.

Zwei Mutanten sind nicht allel, d.h. besitzen eine Mutation in 2 unterschiedlichen Genen, wenn sich die Mutationen in der diploiden Zelle komplementieren und ein Wildtyp Phänotyp resultiert.

### Verwendete Stämme:

| | |
|---|---|
| BMY3-9C (MATα leu2-3,-112 ura3-52 ngd29) | DSM 7193 |
| BMY8-12A (MATa trp1-289^{a} his3-Δ1 ngd62) | DSM 7157 |
| BMY13-1C (MATα ura3-52 leu2-3,-112 his3-Δ1 mnn9) | DSM 7159 |
| BMY13-7B (MATa leu2-3,-112 his3-Δ1 mnn9) | DSM 7158 |
| BMY12-20D(MATα leu2 ura3 his4 ngd62) | DSM 7160 |
| N-BMY3-9C (MATα leu2-3,-112 ura3-52 ngd29) | DSM 7341 |
| N-BMY13-1C (MATα ura3-52 leu2-3,-112 his3-Δ1 mnn9) | DSM 7339 |
| N-BMY12-20D(MATα leu2 ura3 his4 ngd62) | DSM 7340 |

| Kreuzungpartner | | Phänotyp der Haploiden | Selektion Diploide | Phänotyp der Diploiden |
|---|---|---|---|---|
| MATα | MATa | | | |
| BMY3-9C x BMY8-12A | | ngd29xngd62 | his leu | Wildtyp |
| BMY3-9C x BMY13-7B | | ngd29xmnn9 | his ura | Wildtyp |
| BMY13-1C x BMY8-12A | | mnn9xngd62 | trp ura | Wildtyp |
| BMY12-20D x BMY13-7B | | ngd62xmnn9 | his | Wildtyp |
| N-BMY3-9C x BMY8-12A | | ngd29xngd62 | his leu | Wildtyp |
| N-BMY3-9C x BMY13-7B | | ngd29xmnn9 | his ura | Wildtyp |
| N-BMY13-1C x BMY8-12A | | mnn9xngd62 | trp ura | Wildtyp |
| N-BMY12-20D x BMY13-7B | | ngd62xmnn9 | his | Wildtyp |

SC = synthetisches Komplettmedium (0,67% Yeast Nitrogen Base ohne Aminosäuren, Difco; 2% Glucose; 1,5% Agar plus Zusätze: 20 mg/l Trp, His, Arg, Met; 30 mg/l Leu, Ile, Lys; 50 mg/l Phe; 100 mg/l Glu, Asp; 400 mg/l Val, Thr, Ser sowie 20 mg/l Adenin und Uracil; Die Aminosäuren Ura, His und Trp wurden, wie in der Tabelle in der Spalte Selektion der Diploide, angegeben in den einzelnen Minimalmedien weggelassen.

### Ergebnis:

Die Mutanten ngd29 und ngd62 unterscheiden sich untereinander und sind unterschiedlich zu mnn9 (nichtallel).

### Beispiel 7

### Isolierung von GOD und GOD(His)₄ aus Wildtyp und hyperglycosylierungsdefekten Hefestämmen

### 7.1 Isolierung von GOD-(His)4 mittels Metallchelatchromatographie

Mit diesem Isolierverfahren wurde die GOD Variante GOD(His)4 aus dem Kulturfiltrat von BMY3-9A/GOD-(His)4-Zellen und BMy12-20D/GOD-(His)4-Zellen (hyperglycosylierungsdefekte Wirtsstämme) isoliert.

Das Kulturfiltrat wurde mit Natronlauge auf pH 7,5 titriert und auf eine mit 10 mmol/l Kaliumphosphatpuffer, pH 7,5, äquilibrierte NTA-Säule aufgetragen (Säulenvolumen 25 ml; NTA-Gel der Firma Diagen, Düsseldorf; Hochuli, E. et al., J. Chromatography 411 (1987) 177-184; Hochuli, E. et al., Biotechnology 6 (1988) 1321-1325). Die Säule wurde mit 5 - 10 Säulenvolumen 1 mol/l Natriumchlorid in 10 mmol/l Kaliumphosphatpuffer, pH 7,5, und mit 5 - 10 Säulenvolumen 10 mmol/l Kaliumphosphatpuffer, pH 7,5, nachgewaschen. Danach wurde das GOD-(His)₄ Enzym mit 0,1 mol/l Imidazol in Äquilibrierungspuffer, pH 7,5, eluiert und die GOD(His)4 haltigen Fraktionen (gelb) gegen 10 mmol/l Kaliumphosphatpuffer, pH 7,5, dialysiert.

### 7.2 Isolierung von GOD und GOD Varianten durch Ionenaustauschchromatographie an Q-Sepharose ff nach vorheriger Konzentrierung und Dialyse

Nach diesem Verfahren wurde native GOD und hyperglycosylierte GOD aufgereinigt.

100 ml sterilfiltriertes Kulturfiltrat wurden mit 33 g festem Ammoniumsulfat (AS-Sättigungskonzentration 55%) unter langsamen Rühren versetzt, die präzipitierten Proteine nach 1 - 2 Std. Inkubation bei Raumtemperatur abzentrifugiert, in 25 ml 25 mmol/l Kaliumphosphatpuffer, pH 7,5, gelöst und gegen den gleichen Puffer dialysiert (4 X 10 l, 24 Std., 4°C).

Danach wurde das Dialysat auf eine mit 25 mmol/l Kaliumphosphat-Puffer, pH 7,5, äquilibrierte Q-Sepharose ff Säule (Säulenvolumen 12 ml) aufgetragen und mit 5 - 10 Säulenvolumen Äquilibrierungspuffer nachgewaschen. Die gebundenen GOD Enzyme wurden durch einen Gradienten von 0 - 1 mol/l KCl in Äquilibrierungspuffer (ca. 10 Säulenvolumen) eluiert und die GOD-haltigen (gelben) Fraktionen vereinigt.

### Beispiel 8

### Biochemische Charakterisierung der isolierten GOD Enzyme

### 8.1 Bestimmung der spezifischen GOD Aktivität

Die Bestimmung der GOD Aktivität erfolgt, wie in dem Abschnitt allgemeine Methoden beschrieben.

### Spezifische Aktivität von GOD und GOD-(His)4 exprimiert in A. niger, S. cerevisiae (Wildtyp) und S. cerevisiae (hyperglycosylierungsdefekte Mutanten)

| Enzym | Organismus/ Glycosylierung | spez. Aktivität (U/mg Protein) | spez. Aktivität (U/mg Enzym) |
|---|---|---|---|
| GOD | (A. niger) | 225 | 195 |
| GOD | (WT) | 230 | 69 |
| GOD | (ngd29) | 228 | 196 |
| GOD | (ngd62) | 213 | 220 |
| GOD-(His)4 | (WT) | 220 | 68 |
| GOD-(His)4 | (ngd29) | 223 | 200 |
| GOD-(His)4 | (ngd62) | 230 | 225 |
| A. niger, GOD aus A. niger, Reinheit II (Boehringer Mannheim) WT, S. cerevisiae Wildtyp ngd29, S. cerevisiae hyperglycosylierungsdefekte ngd29 Mutante ngd62, S. cerevisiae hyperglycosylierungsdefekte ngd62 Mutante | | | |

### 8.2 Bestimmung des Molekulargewichts durch SDS-Polyacrylamid-Gelelektrophorese (SDS-PAGE)

Die gereinigten GOD Enzyme wurden mit 1/5 Volumen 5xSDS-Probenpuffer (1xSDS-Probenpuffer: 50 mmol/l Tris-HCl, pH 6,8, 1% SDS, 1% Mercaptoethanol, 10% Glycerin, 0,001% Bromphenolblau) versetzt und 5 min bei 95°C inkubiert. Danach wurden die Proteine durch SDS-PAGE aufgetrennt (Laemmli, U.K., Nature 227 (1970) 680-685) und mit Coomassie Brilliant Blue R Farbstoff angefärbt.

### Molekulargewicht/Untereinheit nach SDS-PAGE von GOD und GOD (His)₄ exprimiert in A. niger, S. cerevisiae (Wildtyp) und S. cerevisiae hyperglycosylierungsdefekte Mutanten)

| Enzym | Organismus/ Glycosylierung | Molekulargewicht/Untereinheit (kDa) |
|---|---|---|
| GOD | (A. niger) | ca. 80 |
| GOD | (WT) | 80 - 140 |
| GOD | (ngd29) | ca. 80 |
| GOD | (ngd62) | ca. 80 |
| GOD-(His)4 | (WT) | 80 - 140 |
| GOD-(His)4 | (ngd29) | ca. 80 |
| GOD-(His)4 | (ngd62) | ca. 80 |
| A. niger, GOD aus A. niger, Reinheit II (Boehringer Mannheim) WT, S. cerevisiae Wildtyp ngd29, S. cerevisiae hyperglycosylierungsdefekte ngd29 Mutante ngd62, S. cerevisiae hyperglycosylierungsdefekte ngd62 Mutante | | |

### 8.3 Bestimmung des Kohlenhydratanteils (Anthron-Reaktion)

Der Kohlenhydratanteil der GOD Enzyme aus unterschiedlichen Organismen bzw. Hefestämmen wurde in Anlehnung an die Methode von Ashwell, G. (Methods Enzymol. 3 (1957) 84) bestimmt.

Hierzu werden 0,5 ml gereinigtes GOD Enzym (Konzentration 20 - 100 U/ml in H₂O) mit 5 ml Anthron-Reagenz gemischt, die Lösung 5 Minuten bei 25°C inkubiert und danach 15 Minuten im siedenden Wasserbad erhitzt. Nach Abkühlung der Probe auf 25°C wird die Extinktion bei 630 nm gegen einen Reagentienleerwert bestimmt. Mittels einer simultan erstellten Mannose-Eichkurve mit Mannose-Standardlösungen von 5, 25, 75 und 100 *µ*g/ml Mannose wird der Kohlenhydratanteil der GOD Probe ermittelt.

### Herstellung des Anthron-Reagenzes:

66 ml konzentrierte Schwefelsäure werden vorsichtig mit 34 ml Wasser verdünnt. Nach Abkühlung auf 80°C werden 50 mg Anthron und 1 g Thioharnstoff in der Schwefelsäure gelöst. Das Anthron-Reagenz ist zwei Wochen bei 4°C haltbar.

### Kohlenhydratanteil von GOD und GOD-(His)₄ exprimiert in A. niger, S. cerevisiae (Wildtyp) und S. cerevisiae (hyperglycosylierungsdefekte Mutanten)

| Enzym | Organismus/ Glycosylierung | Kohlenhydratanteil (%) (bezogen auf Protein) |
|---|---|---|
| GOD | (A. niger) | 13 |
| GOD | (WT) | 71 |
| GOD | (ngd29) | 12,5 |
| GOD | (ngd62) | 13 |
| GOD-(His)4 | (WT) | 65 |
| GOD-(His)4 | (ngd29) | 11 |
| GOD-(His)4 | (ngd62) | 12 |
| A. niger, GOD aus A. niger, Reinheit II (Boehringer Mannheim) WT, S. cerevisiae Wildtyp ngd29, S. cerevisiae hyperglycosylierungsdefekte ngd29 Mutante ngd62, S. cerevisiae hyperglycosylierungsdefekte ngd62 Mutante | | |

### Publikationen:

Bekkers, A.C.A.P.A.; Franken, P.A.F.; Van den Bergh, C.J.; Verbakel, J.M.A.; Verheij, H.M.; De Haas,G.H.: The use of genetic engineering to obtain efficient production of porcine pancreatic phospholipase A2 by Saccharomyces cerevisiae. Biochim. Biophys. Acta 1089, 345-351 (1991).

Ballou, L.; Cohen, R.E.; Ballou,C.E.: Saccharomyces cerevisiae mutants that make mannoproteins with a truncated carbohydrate outer chain. J. Biol. Chem. 255, 5986-5991 (1980).

Ballou, C.E.: Yeast cell wall and cell surface. In: Strathern, J.N.; Jones, E.W.; Broach, J.R. (eds.), The Molecular Biology of the Yeast Saccharomyces, Metabolism and Gene Expression, Cold Spring Harbor Laboratory, New York, pp. 335-360 (1982).

Ballou, L.; Alvarado, E.; Tsai, P.; Dell, A; Ballou, C.E.: Protein glycosylation defects in the Saccharomyces cerevisiae mnn7 mutant class. J. Biol. Chem. 264, 11857-11864 (1989).

Ballou, C.E.: Isolation characterization, and properties of Saccharomyces cerevisiae mmn mutants with nonconditional protein glycosylation defects. Methods Enzymol. 185, 440-470 (1990).

Ballou, L.; Hitzeman, R.A.; Lewis, M.S.; Ballou, C.S.: Vanadate-resistant yeast mutants are defective in protein glycosylation. Proc. Natl. Acad. Sci. 88, 3209-3212 (1991).

Beggs, J.D.: Transformation of yeast by a replicating hybrid plasmid. Nature 275, 104-109 (1978).

Carlson, M.; Taussig, R.; Kustu, S.; Botstein, D.: The secreted form of invertase in Saccharomyces cerevisiae is synthesized from mRNA encoding a signal sequence. Mol. Cell. Biol. 3, 439-447 (1983).

Ciriacy, M.: Genetics of alcohol dehydrogenase in Saccharomyces cerevisiae. I. Isolation and genetic analysis of adh-mutants. Mut. Res. 29, 315-326 (1975).

De Baetselier, A.; Vasavada, A.; Dohet, P.; Ha-Ti, V.; De Beukelaer, M.; Erpicum, T.; De Clerk, L.; Hanotier, J.; Rosenberg, S.: Fermentation of yeast producing A. niger glucose oxidase: scale up, purification and characterization of the recombinant enzyme. Biotechnology 9, 559-561 (1991),

Delorme, E.: Transformation of Saccharomyces cerevisiae by electroporation. Applied and Environmental Microbiology 55, 2242-2246 (1989).

Dijken, J.P. van; Veenhuis M.: Cytochemical localization of glucose oxidase in peroxisomes of Aspergillus niger. European J. Appl. Microbiol. Biotechnol. 9, 275 - 283 (1980) Erhart, E.; Hollenberg, C.P.: The presence of a defective LEU2 gene on 2*µ*DNA recombinant plasmids of Saccharomyces cerevisiae is responsible for curing and high copy number. J. Bacteriol. 156, 625-635 (1983).

Frederick, K.R.; Tung, J.; Emerick, R.S.; Masiarz, F.R.; Chamberlain, S.H.; Vasavada, A.; Rosenberg, S.; Chakraborty, S.; Schopter, L.M.; Massey, N.: Glucose oxidase from Aspergillus niger. J. Biol. Chem. 265, 3793-3802 (1990).

Hadwick, K.G.; Lewis, M.J.; Semenza, J.; Dean, N.; Pelham, H.R.B.: ERD1, a yeast gene required for the retention of luminal endoplasmic reticulum proteins, affects glycoprotein processing in the Golgi apparatus. EMBO J. 9, 623-630 (1990).

Haselbeck, A.; Hösel, W.: Studies on the effect of the incubation conditions, various detergents and protein concentration on the enzymatic activity of N-glycosidase F (glycopeptidase F) and endoglycosidase F. Topics in Biochemistry 8, 1- 4 (1988).

Hochuli, E.; Doebeli, H.; Schacher, A.: New metal chelate adsorbent selective for proteins and peptides containing neighbouring histidine residues. J. Chromatography 411, 177-184 (1987).

Hochuli, E.; Bannwarth, W.; Doebeli, H.; Genz, R.; Stueber, D.: Genetic approach to facilitate purification of recombinant proteins with a novel metal chelate adsobent. Biotechnology 6, 1321-1325 (1988).

Huffaker, T.C.; Robbins, P.W.: Yeast mutants deficient in protein glycosylation. Proc. Natl. Acad. Sci. 80, 7466-7470 (1983).

Innis, M.A.: Glycosylation of heterologous proteins in Saccharomyces cerevisiae. In: Barr, P.J.; Brake, A.J.; Valenzuela, P. (eds.), Yeast genetic engineering, Butterworths, Stoneham, Mass, pp. 233-246 (1989).

Ito, H.; Jukuda, A.; Murata, K.; Kimura, A: Transformation of intact yeast cells treated with alkali cations. J. Bacteriol. 153, 163-168 (1983).

Kopetzki, E.; Buckel, P.; Schumacher, G.: Cloning and characterization of baker's yeast alpha glucosidase: overexpression in a yeast strain devoid of vacuolar proteinases. Yeast 5, 11-24 (1989).

Kornfeld, R.; Kornfeld, S.: Assembly of asparagine-linked oligosaccharides. Ann. Rev. Biochem. 54, 631-664 (1985).

Kukuruzinska, M.A.; Bergh, M.L.E.; Jackson, B.J.: Protein glycosylation in yeast. Ann. Rev. Biochem. 56, 915-944 (1987).

Kriechbaum, M.; Heilmann, H,J.; Wientjes, F.J.; Hahn, M.; Jany, K.-D.; Gassen, H.G.; Sharif, F.; Alaeddinoglu, G.: Clonig and DNA sequence analysis of the glucose oxidase gene from Aspergillus niger NRRL-3. FEBS Lett. 255, 63-66 (1989).

Laemmli, U.K.: Cleavage of structural proteins during the assembly of the head of bacteriophage T4. Nature 227, 680-685 (1970).

Maniatis, T. et al., In: Molecular cloning: A laboratory manual. Cold Spring Harbor Laboratory Press, Cold Spring Harbor, New York, (1989).

Moir, D.T.: Yeast mutants with increased secretion efficiency. In: Barr, P.J.; Brake, A.J.; Valenzuela, P. (eds.), Yeast genetic engineering, Butterworths, Stoneham, Mass, pp. 215-231 (1989).

Mullis, K.B.; Faloona, F.A.: Specific synthesis of DNA in vitro via a polymerase-catalyzed chain reaction. Methods Enzymol. 155, 355-350 (1987).

Nakano, A.; Muramatsu, M.: A novel GTP-binding protein, Sarp1, is involved in transport from the endoplasmic reticulum to the Golgi apparatus. J. Cell. Biol. 109, 2677-2691 (1989).

Newman, A.P.; Ferro-Novick, S.: Characterization of new mutants in the early part of the yeast secretory pathway isolated by [3H]mannose suicide selection. J. Cell. Biol. 105, 1587-1594 (1987).

Novick, P.; Field, C.; Schekman, R.: Identification of 23 complementation groups required for post-translational events in the yeast secretory pathway. Cell 21, 205-215 (1980).

Paulson, C.P.: Glycoproteins: what are the sugar chains for? TIBS 14, 272-276 (1989).

Rudolph, H.K., Antebi, A.; Fink, G.R.; Buckley, C.M.; Dorman, T.E.; LeVitre, J.; Davidow, L.S.; Mao, J.; Moir, D.T.: The yeast secretory pathway is perturbed by mutations in PMR1, a member of a Ca2+ ATPase family. Cell 58, 133-145 (1989).

Reddy, V.A.; Johnson, R.S.; Biemann, K.; Williams, R.S.; Ziegler, F.D.; Trimble, R.B.; Maley, F.: Characterization of glycosylation sites in yeast external invertase. I. N-linked oligosaccharide content of the individual sequons. J. Biol. Chem. 263, 6978-6985 (1988).

Runge, K.W.; Robbins, P.W.: Saccharomyces cerevisiae mutants in the early stages of protein glycosylation. In: Bonventre, P.F.; Morello, J.A.M.; Silver, S.D.; Wu, H.C. (eds.), Microbiology-1986. American Society for Microbiology, Washington, D.C. pp. 312-316 (1986).

Schekman, R.; Novick, P.: The secretory process and yeast cell-surface assembly. In: Strathern, J.N.; Jones, E.W.; Broach, J.R. (eds.), The Molecular Biology of the Yeast Saccharomyces, Metabolism and Gene Expression, Cold Spring Harbor Laboratory, New York, pp. 361-398 (1982).

Schmitt, H.D.; Wagner, P.; Pfaff, E.; Gallwitz, D.: The ras-related YPT1 gene product in yeast: a GTP-binding protein that might be involved in microtubule organization. Cell 47, 401-412 (1986).

Schmitt, H.D.; Puzichia, M.; Gallwitz, D.: Study of a temperature-sensitive mutant of the ras-related YPT1 gene product in yeast suggests a role in the regulation of intracellular calcium. Cell 53, 635-647 (1988).

Sherman, F.; Fink, G.R.; Hicks, J.B.: Methods in Yeast Genetics: A Laboratory Manual, Cold Spring Harbor Laboratory, Cold Spring Harbor, New York, (1981).

Tanner, W.; Lehle, L.: Protein glycosylation in yeast. Biochim. Biophys. Acta 906, 81-99 (1987).

Warren, C.E.: Glycosylation- considerations for protein engineering. BFE 7, 392-395 (1990).

Whittington, H.; Kerry-Williams, S.; Bidgood, K.; Dodsworth, N. Peberdy, J.; Dobson, M.; Binchliffe, E.; Ballance, D.J.: Expression of the Aspergillus niger glucose oxidase gene in A. niger, A. nidulans and Saccharomyces cerevisiae. Curr. Genet. 18, 531-536 (1990).

Zamenhof, S.: Preparation and assay of deoxyribonucleic acid from animal tissue. Methods Enzymol. 3, 696-704 (1957).

Ziegler, F.D.; Maley, F.; Trimble, R.B.: Characterization of glycosylation sites in yeast external invertase. II. Location of the endo-betta-N-acetylglucosaminidase H-resistant sequons. J. Biol. Chem. 263, 6978-6985 (1988).

### SEQUENCE LISTING

(iii) NUMBER OF SEQUENCES: 10
(2) INFORMATION FOR SEQ ID NO:1:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:1:
   ATTTCTCCTT ATTGCGCGCT T                                                  21
(2) INFORMATION FOR SEQ ID NO:2:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (xi) SEQUENCE DESCRIPTION: SEQ ID NO:2:
   TCTATTCAGC TGTCGACATA GATCTTATGT AATTTAGTTA CGCTTGAC 48
(2) INFORMATION FOR SEQ ID NO:3:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (xi) SEQUENCE DESCRIPTION: SEQ ID NO:3:
   AGATCTATGT CGACAGCTGA ATAGATAAAA TTAGTGCGGA CTTTTTTTTA 50
(2) INFORMATION FOR SEQ ID NO:4:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:4:
   GTCATTTGTA AAGTAAAATT CCAA 24
(2) INFORMATION FOR SEQ ID NO:5:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ-ID NO:5:
   GCCCGGTACC AGATCTATGC AGACTCTCCT TGTGAGCT 38
(2) INFORMATION FOR SEQ ID NO:6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:6:
   TCTAGAACTA GTGGATCCCC C 21
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:7:
   GCCGGCGAAC GTGGCGAGAA 20
(2) INFORMATION FOR SEQ ID NO:8:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:8:
   ATATATCAGC TGTCACTGCA TGCTAGCATA ATCTTCCAAG ATAGC 45
(2) INFORMATION FOR SEQ ID NO:9:
   (i) SEQUENCE CHARACTERISTICS:
      (A) LENGTH: 21 base pairs
      (B) TYPE: nucleic acid
      (C) STRANDEDNESS: singie
      (D) TOPOLOGY: linear
   (xi) SEQUENCE DESCRIPTION: SEQ ID NO:9:
   CAGCACCACC ACCACTGACA G 21
(2) INFORMATION FOR SEQ ID NO:10:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:10:
   CTGTCAGTGG TGGTGGTGCT GCATG                                    25

## Patentansprüche

1. Hefemutanten mit Defekten in der N-Glycosylierung, **dadurch gekennzeichnet, daß** diese Hefemutanten Proteine in hypoglycosylierter Form ohne Kontamination mit "outer chain" Glycosylierung (GlcNac₂, Man₅₀₋₁₀₀) exprimieren, erhältlich durch [³H]-Mannose Suizidselektion, Einführung einer oder mehrerer selektiver Marker (Auxotrophien und/oder Resistenzen), Auswahl derjenigen Stämme, welche nach Transformation mit dem Plasmid DSM 7038, enthaltend das Glucoseoxidasestrukturgen aus dem Hefestamm ATCC 9029, und Fermentation in Vollmedium mit 2% Hefeextrakt, 4% Bactopepton, Difco, 0,1 mol/l Phosphatpuffer, pH 7,0, 1% Fructose und 6% Maltose nach 3-4 Tagen Inkubation unter Schütteln in das Medium 10 mg/l Glucoseoxidase oder mehr sezernieren und allel sind zu Saccharomyces cerevisiae DSM 7042, DSM 7338, DSM 7160 und/oder DSM 7340.

2. Hefe-Mutanten DSM 7042, DSM 7338, DSM 7160 und DSM 7340.

3. Verfahren zur Herstellung von Hefe-Mutanten gemäß den Ansprüchen 1 und 2 durch [³H]-Mannose Suizidselektion, **gekennzeichnet durch** Einführung einer oder mehrerer selektiver Marker (Auxotrophien und/oder Resistenzen), Auswahl derjenigen Stämme, welche nach Transformation mit dem Plasmid DSM 7038, enthaltend das Glucoseoxidasestrukturgen aus dem Hefestamm ATCC 9029, und Fermentation in Vollmedium mit 2% Hefeextrakt, 4% Bactopepton, Difco, 0,1 mol/l Phosphatpuffer pH 7,0, 1% Fructose und 6% Maltose nach 3-4 Tagen Inkubation unter Schütteln in das Medium 10 mg/l Glucoseoxidase oder mehr sezernieren und allel sind zu Saccharomyces cerevisiae DSM 7042, DSM 7338, DSM 7160 und/oder DSM 7340.

4. Verfahren nach Anspruch 3 zur Herstellung von Saccharomyces cerevisiae DSM 7042, DSM 7338, DSM 7160 und/oder DSM 7340.

5. Verwendung der Hefe-Mutanten nach Anspruch 1 oder 2 als Wirtsorganismus zur homologen und heterologen Expression von Proteinen.

6. Verfahren zur Herstellung eines rekombinanten Proteins in hypoglycosylierter Form ohne Kontamination mit "outer chain" Glycosylierung (GlcNac₂, Man₅₀₋₁₀₀) durch Transformation einer Hefemutante nach Anspruch 1 oder 2 durch eine weitere rekombinante DNA, welche für das genannte rekombinante Protein codiert, Fermentation der genannten Mutante und Isolierung des Proteins aus den Zellen oder dem Kulturüberstand.

## Claims

1. Yeast mutants with defects in N-glycosylation, wherein these yeast mutants express proteins in a hypoglycosylated form without contamination by outer chain glycosylation (GlcNac₂, Man₅₀₋₁₀₀) obtainable by [³H]-mannose suicide selection, introducing one or several selective markers (auxotrophies and/or resistances), selecting those strains which secrete 10 mg/l glucose oxidase or more into the medium after transformation with the plasmid DSM 7038 containing the glucose oxidase structural gene from the yeast strain ATCC 9029 and fermentation in complete medium containing 2 % yeast extract, 4 % bactopeptone, Difco, 0.1 mol/l phosphate buffer, pH 7.0, 1 % fructose and 6 % maltose after 3-4 days incubation while shaking and are allelic to Saccharomyces cerevisiae DSM 7042, DSM 7338, DSM 7160 and/or DSM 7340.

2. Yeast mutants DMS 7042, DSM 7338, DSM 7160 and DSM 7340.

3. Process for the production of yeast mutants as claimed in claims 1 and 2 by [³H]-mannose suicide selection **characterized by** introducing one or several selective markers (auxotrophies and/or resistances), selecting those strains which secrete 10 mg/l glucose oxidase or more into the medium after transformation with the plasmid DSM 7038 containing the glucose oxidase structural gene from the yeast strain ATCC 9029 and fermentation in complete medium containing 2 % yeast extract, 4 % bactopeptone, Difco, 0.1 mol/1 phosphate buffer, pH 7.0, 1 % fructose and 6 % maltose after 3-4 days incubation while shaking and are allelic to Saccharomyces cerevisiae DSM 7042, DSM 7338, DSM 7160 and/or DSM 7340.

4. Process as claimed in claim 3 for the production of Saccharomyces cerevisiae DSM 7042, DSM 7338, DSM 7160 and/or DSM 7340.

5. Use of the yeast mutants as claimed in claim 1 or 2 as a host organism for the homologous and heterologous expression of proteins.

6. Process for the production of a recombinant protein in a hypoglycosylated form without contamination by outer chain glycosylation (GlcNac₂, Man₅₀₋₁₀₀) by transforming a yeast mutant as claimed in claim 1 or 2 by a further recombinant DNA which codes for the said recombinant protein, fermenting the said mutant and isolating the protein from the cells or the culture supernatant.

## Revendications

1. Mutants de levure présentant des défauts lors de la N-glycosylation, **caractérisés en ce que** ces mutants de levure expriment des protéines sous forme hypoglycosylée sans contamination par une glycosylation "en chaîne extérieure" (GlcNac₂, Man₅₀₋₁₀₀), pouvant être obtenus par sélection suicide de [³H]-mannose, introduction d'un ou plusieurs marqueurs sélectifs (auxotrophies et/ou résistances), sélection des souches qui, après transformation par le plasmide DSM 7038, qui contient le gène de structure de la glucose-oxydase dérivé de la souche de levure ATCC 9029, et fermentation dans un milieu complet contenant 2 % d'extrait de levure, 4 % de bactopeptone, Difco, 0,1 mol/l d'un tampon phosphate, pH 7,0, 1 % de fructose et 6 % de maltose, sécrètent après 3-4 jours d'incubation en présence de secousses dans le milieu 10 mg/I de glucose oxydase ou plus, et sont alléliques avec Saccharomyces cerevisiae DSM 7042, DSM 7338, DSM 7160 et/ou DSM 7340.

2. Mutants de levure DSM 7042, DSM 7338, DSM 7160 et DSM 7340.

3. Procédé de préparation de mutants de levure selon les revendications 1 et 2, par sélection suicide de [³H]-mannose, **caractérisé par** l'introduction d'un ou plusieurs marqueurs sélectifs (auxotrophies et/ou résistances), la sélection des souches qui, après transformation par le plasmide DSM 7038, qui contient le gène de structure de la glucose-oxydase provenant de la souche de levure ATCC 9029, et fermentation dans un milieu complet contenant 2 % d'extrait de levure, 4 % de bactopeptone, Difco, 0,1 mol/l d'un tampon phosphate pH 7,0, 1 % de fructose et 6 % de maltose, sécrètent au bout de 3-4 jours d'incubation en présence de secousses dans le milieu 10 mg/l de glucose-oxydase ou plus, et sont alléliques avec Saccharomyces cerevisiae DSM 7042, DSM 7338, DSM 7160 et/ou DSM 7340.

4. Procédé selon la revendication 3 pour préparer Saccharomyces cerevisiae DSM 7042, DSM 7338, DSM 7160 et/ou DSM 7340.

5. Utilisation des mutants de levure selon la revendication 1 ou 2 en tant qu'organisme hôte pour l'expression homologue et hétérologue de protéines.

6. Procédé de préparation d'une protéine recombinante sous forme hypoglycosylée sans contamination par une glycosylation "en chaîne extérieure" (GlcNac₂, Man₅₀₋₁₀₀), par transformation d'un mutant de levure selon la revendication 1 ou 2 par un ADN recombinant supplémentaire codant pour la protéine recombinante mentionnée, fermentation des mutants mentionnés, et isolement de la protéine à partir des cellules ou du surnageant de culture.
